# EUROPEAN PATENT SPECIFICATION

(11) **EP 2 616 471 B1**
(45) Date of publication and mention of the grant of the patent: **31.12.2014**
(21) Application number: 11761206.9
(22) Date of filing: 13.09.2011
(51) Int. Cl.: C07D 495/04

(54) **INHIBITORS OF NOTUM PECTINACETYLESTERASE AND METHODS OF THEIR USE**
INHIBITOREN VON NOTUM PECTINACETYLESTERASE UND VERFAHREN ZU IHRER VERWENDUNG
INHIBITEURS DE LA NOTUM PECTINE ACÉTYLESTÉRASE ET PROCÉDÉS POUR LEUR UTILISATION

(30) Priority: 14.09.2010 US 382526 P
(43) Date of publication of application: 24.07.2013
(73) Proprietor: Lexicon Pharmaceuticals, Inc., The Woodlands, TX 77381 (US)
(72) Inventor: BARBOSA, Joseph, Lambertville New Jersey 08530 (US); CARSON, Kenneth Gordon, Princeton New Jersey 08540 (US); GARDYAN, Michael Walter, Feasterville Pennsylvania 19053 (US); HE, Wei, Beijing 100084 (CN); LOMBARDO, Victoria, Belle Mead New Jersey 08502 (US); PABBA, Praveen, Pennington New Jersey 08534 (US); TARVER, James Jr., Morrisville Pennsylvania 19067 (US)
(74) Representative: Hewson, Timothy John
(86) International application number: PCT/US2011/051421
(87) International publication number: WO 2012/037141

(56) References cited:
- WO-A2-2009/001214
- ROBBA M ET AL: "THIENOPYRIMIDINES-II ETUDE DE LA THIENO Ú3.2-D 3/4 PYRIMIDINE ET DE QUELQUES DERIVES", TETRAHEDRON, ELSEVIER SCIENCE PUBLISHERS, AMSTERDAM, NL, vol. 27, no. 2, 1 January 1971 (1971-01-01), pages 487-499, XP000673526, ISSN: 0040-4020, DOI: 10.1016/S0040-4020(01)90718-5
- HUI-FANG GUO ET AL: "Substituted Benzothiophene or Benzofuran Derivatives as a Novel Class of Bone Morphogenetic Protein-2 Up-Regulators: Synthesis, Structure-Activity Relationships, and Preventive Bone Loss Efficacies in Senescence Accelerated Mice (SAMP6) and Ovariectomized Rats", JOURNAL OF MEDICINAL CHEMISTRY, vol. 53, no. 4, 25 February 2010 (2010-02-25), pages 1819-1829, XP55010758, ISSN: 0022-2623, DOI: 10.1021/jm901685n cited in the application
- KATADA J ET AL: "Cytotoxic effects of NSL-1406, a new thienopyrimidine derivative, on leukocytes and osteoclasts", BIOORGANIC & MEDICINAL CHEMISTRY LETTERS, PERGAMON, ELSEVIER SCIENCE, GB, vol. 9, no. 6, 22 March 1999 (1999-03-22), pages 797-802, XP004160476, ISSN: 0960-894X, DOI: 10.1016/S0960-894X(99)00088-8
- NI, YIKE ET AL: "Identification and SAR of a new series of thieno[3,2-d]pyrimidines as Tpl2 kinase inhibitors", BIOORGANIC & MEDICINAL CHEMISTRY LETTERS , 21(19), 5952-5956 CODEN: BMCLE8; ISSN: 0960-894X, 26 July 2011 (2011-07-26), XP002662391,

## Description

### 1. FIELD OF THE INVENTION

This invention relates small molecule inhibitors of Notum Pectinacetylesterase, compositions comprising them, and methods of their use.

### 2. BACKGROUND OF THE INVENTION

Bone health depends on the coordinated activities of bone forming osteoblasts and bone resorbing osteoclasts. "Bone turnover reflects a balance between these anabolic and catabolic cellular functions and ensures that the mature skeleton can repair itself when damaged and sustain its endocrine function by release of minerals such as calcium and phosphorous into the circulation." Allen, J.G. et al., J. Med. Chem., 53 (June 10, 2010), pp. 4332 - 4353, 4332. Many disease states alter this balance, resulting in increased or decreased bone mass or changes in bone quality. Gradual loss of bone mineral density is known as osteopenia; severe loss of bone is known as osteoporosis. *Id.*

The current standard of care for the treatment and prevention of osteoporosis utilizes the bisphosphonate class of oral, small molecule antiresportives. *Id.* at 4333. Zoledronic acid, raloxifene, calcium, and vitamin D supplements are also typically used in the osteoporosis treatment. *Id.* While antiresporptive agents can help prevent bone loss, anabolic agents "are capable of increasing bone mass to a greater degree ... and also have the capacity to improve bone quality and increase bone strength." Guo, H., et al., J. Med. Chem., 53 (February 25, 2010), pp. 1819 - 1829, 1819. In the United States, human PTH is the only FDA-approved anabolic agent. *Id.;* Allen at 4333. "Because of the paucity of available anabolic agents for osteoporosis treatment, there is an urgent need to develop small molecular compounds to treat this disease that are nontoxic, cost-effective, and easy to administer." Guo, at 1819.

"Although the development of pharmacological agents that stimulate bone formation is less advanced compared to antiresporptive therapies, several pathways are known to facilitate osteoblast function." Allen at 4338. These pathways include bone morphogenic proteins, transforming growth factor β, parathyroid hormone, insulin-like growth factor, fibroblast growth factor, and wingless-type MMTV integration site (WNT) signaling. *Id.* Guo and coworkers recently reported results concerning the first of these pathways. Guo, *supra.* In particular, they reported that certain substituted benzothiophene and benzofuran compounds enhance bone morphogenic protein 2 expression in mice and rats. Two of the compounds reportedly stimulate bone formation and trabecular connectivity restoration in vivo. *Id.* at 1819.

Another of these pathways is the WNT pathway, which is implicated in a variety of developmental and regenerative processes. Allen at 4340. The pathway is complex, however, and much about it and about how its components affect bone remains unclear. For example, it has been suggested that LRP-5, mutations of which are associated with increased bone mass in humans, and β-catenin, through which canonical WNT signaling occurs, "may not be linked directly via WNT signaling to the control of bone mass." *Id.*

Recent analysis of gene expression data has led to the identification of new targets of WNT signaling. *See, e.g.,* Torisu, Y., et al., Cancer Sci., 99(6):1139-1146, 1143 (2008). One such target is Notum Pectinacetylesterase, also known as NOTUM and LOC174111.

### 3. SUMMARY OF THE INVENTION

This invention encompasses compounds of the formula: 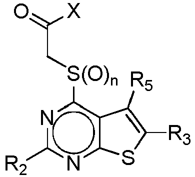 or a pharmaceutically acceptable salt thereof, wherein: X is OR_{1B} or N(R_{1B})₂; each R_{1B} is independently hydrogen or optionally substituted alkyl, aryl, heteroalkyl, or heterocycle; R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle; each R_{2A} is independently hydrogen or optionally substituted alkyl, aryl, heteroalkyl, or heterocycle; R₃ is hydrogen, halo, cyano, -
CO₂R_{3A}, -C(O)N(R_{3A})₂, -SR_{3A}, -OR_{3A}, -N(R_{3A})₂, or optionally substituted alkyl, aryl, heteroalkyl, or heterocycle; each R_{3A} is independently hydrogen or optionally substituted alkyl, aryl, heteroalkyl, or heterocycle; R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle; each R_{5A} is independently hydrogen or optionally substituted alkyl, aryl, heteroalkyl, or heterocycle; and
n is 1 or 2; wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

The invention also encompasses compounds of the formula: 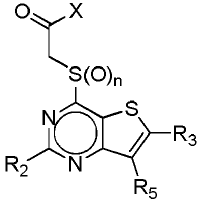 or a pharmaceutically acceptable salt thereof, wherein: X is OR_{1B} or N(R_{1B})₂; each R_{1B} is independently hydrogen or optionally substituted alkyl, aryl, heteroalkyl, or heterocycle; R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle; each R_{2A} is independently hydrogen or optionally substituted alkyl, aryl, heteroalkyl, or heterocycle; R₃ is hydrogen, halo, cyano, -
CO₂R_{3A}, -C(O)N(R_{3A})₂, -SR_{3A}, -OR_{3A}, -N(R_{3A})₂, or optionally substituted alkyl, aryl, heteroalkyl, or heterocycle; each R_{3A} is independently hydrogen or optionally substituted alkyl, aryl, heteroalkyl, or heterocycle; R₄ is hydrogen, halo, or optionally substituted alkyl, aryl, heteroalkyl, or heterocycle; R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle; each R_{5A} is independently hydrogen or optionally substituted alkyl, aryl, heteroalkyl, or heterocycle; and n is 1 or 2; wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

This invention encompasses pharmaceutical compositions comprising the compounds disclosed herein.

The invention also encompasses compounds of: 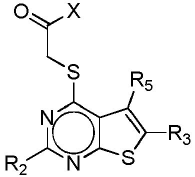 or a pharmaceutically acceptable salt thereof, for use as a medicament for the treatment or management of a disease or disorder characterized by bone loss, wherein: X is OR_{1B} or N(R_{1B})₂; each R_{1B} is independently hydrogen or optionally substituted alkyl, aryl, heteroalkyl, or heterocycle; R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle; each R_{2A} is independently hydrogen or optionally substituted alkyl, aryl, heteroalkyl, or heterocycle; R₃ is hydrogen, halo, cyano, - CO₂R_{3A}, -C(O)N(R_{3A})₂, -SR_{3A}, -OR_{3A}, -N(R_{3A})₂, or optionally substituted alkyl, aryl, heteroalkyl, or heterocycle; each R_{3A} is independently hydrogen or optionally substituted alkyl, aryl, heteroalkyl, or heterocycle; R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle; and each R_{5A} is independently hydrogen or optionally substituted alkyl, aryl, heteroalkyl, or heterocycle; wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

### 4. BRIEF DESCRIPTION OF THE FIGURES

Certain aspects of the invention may be understood with reference to the attached figures.
Figure 1 provides a graphical representation of differences between the cortical thicknesses of various bone sites in NOTUM homozygous knockout mice ("HOM") and those in their wildtype littermates ("WT").
Figure 2 provides a graphical representation of an increase in cortical bone thicknesses observed in both NOTUM homozygous and heterozygous ("HET") knockout mice as compared to their wildtype littermates.
Figure 3 provides a graphical representation of results obtained from femur breaking strength and spine compression tests performed on the bones of male NOTUM homozygous and heterozygous knockout mice and their wildtype littermates.
Figure 4 provides a graphical representation of results obtained from femur breaking strength and spine compression tests performed on the bones of female NOTUM homozygous and heterozygous knockout mice and their wildtype littermates
Figure 5 provides a graphical representation of midshaft femur cortical thickness measurements obtained after 25 days of dosing F1 male hybrid (129 x C57) mice with 1 mg/kg, 8 mg/kg, and 24 mg/kg of the NOTUM inhibitor 2-((5,6-dimethylthieno[2,3-d]pyrimidin-4-yl)thio)acetic acid.

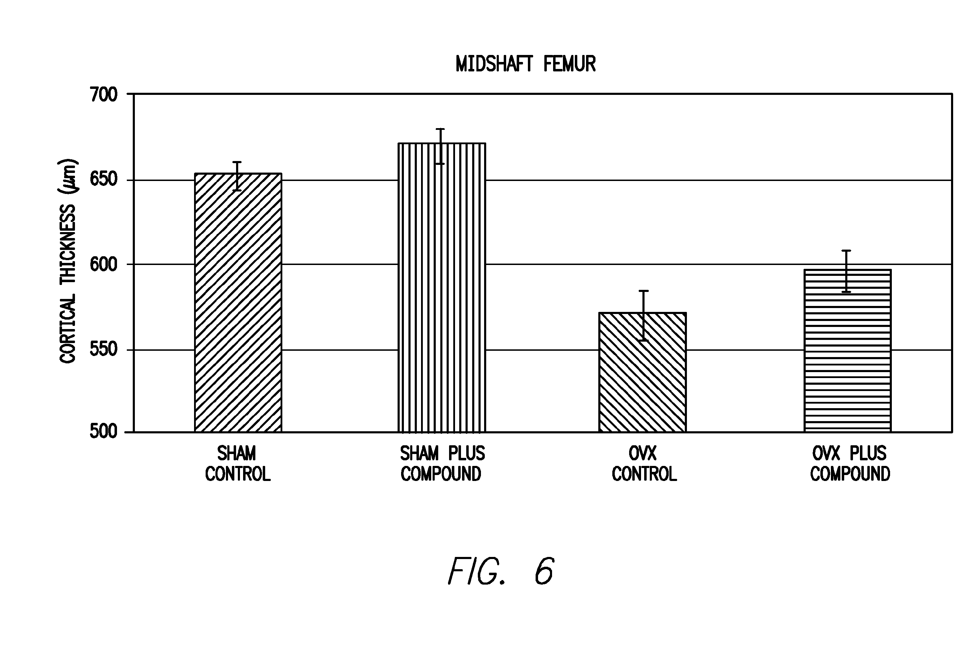

Figure 6 provides a graphical representation of midshaft femur cortical thickness measurements obtained after five weeks of dosing Fischer 344 ovariectomized rats with 2-((5-chloro-6-methylthieno[2,3-d]pyrimidin-4-yl)thio)acetic acid, wherein "SHAM CONTROL" refers to rats that were not ovariectomized and not administered compound; "SHAM PLUS COMPOUND" refers to rats that were not ovariectomized but were administered compound; "OVX CONTROL" refers to rats that were ovariectomized but not administered compound; and "OVX PLUS COMPOUND" refers to rats that were ovariectomized and were administered compound.
Figure 7 provides a graphical representation of midshaft tibia cortical thickness measurements obtained from the same experiment associated with Figure 6.
Figure 8 provides a graphical representation of midshaft femur cortical thickness measurements obtained after 25 days of dosing F1 male hybrid (129 x C57) mice with 3 mg/kg, 10 mg/kg, and 30 mg/kg of the NOTUM inhibitor 2-((6-chloro-7-cyclopropylthieno[3,2-d]pyrimidin-4-yl)thio)acetic acid.
Figure 9 provides a graphical representation of midshaft femur cortical thickness measurements obtained after seven and 18 days of dosing F1 male hybrid (129 x C57) mice with 34 mg/kg of the NOTUM inhibitor 2-((6-chloro-7-methylthieno[3,2-d]pyrimidin-4-yl)thio)acetic acid.

### 5. DETAILED DESCRIPTION OF THE INVENTION

This invention is based, in part, on the discovery that inhibition of NOTUM can affect endocortical bone formation. Particular aspects of the invention are based on studies of mice lacking a functional NOTUM gene ("knockout mice"), on the discovery of compounds that inhibit NOTUM, and on the discovery that such compounds can be used to stimulate cortical bone formation in mice and rats.

### 5.1. Definitions

Unless otherwise indicated, the term "alkenyl" means a straight chain, branched and/or cyclic hydrocarbon having from 2 to 20 *(e.g.,* 2 to 10 or 2 to 6) carbon atoms, and including at least one carbon-carbon double bond. Representative alkenyl moieties include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl and 3-decenyl.

Unless otherwise indicated, the term "alkoxy" means an -O-alkyl group. Examples of alkoxy groups include, but are not limited to, -OCH₃, - OCH₂CH₃, -O(CH₂)₂CH₃, -O(CH₂)₃CH₃, -O(CH₂)₄CH₃, and -O(CH₂)₅CH₃.

Unless otherwise indicated, the term "alkyl" means a straight chain, branched and/or cyclic ("cycloalkyl") hydrocarbon. Non-cyclic alkyl moieties may have from 1 to 20 *(e.g.,* 1 to 10 or 1 to 4) carbon atoms; cyclic alkyl moieties may have from 3-20 *(e.g.,* 3-10 or 3-6) carbon atoms. Alkyl moieties having from 1 to 4 carbons are referred to as "lower alkyl." Examples of alkyl groups include, but are not limited to, methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl and dodecyl. Cycloalkyl moieties may be monocyclic or multicyclic, and examples include cyclopropyl, cyclobutyl, cyclopentyl, cyclohexyl, and adamantyl. Additional examples of alkyl moieties have linear, branched and/or cyclic portions (*e.g*., 1-ethyl-4-methyl-cyclohexyl). The term "alkyl" includes saturated hydrocarbons as well as alkenyl and alkynyl moieties.

Unless otherwise indicated, the term "alkylaryl" or "alkyl-aryl" means an alkyl moiety bound to an aryl moiety.

Unless otherwise indicated, the term "alkylheteroaryl" or "alkyl-heteroaryl" means an alkyl moiety bound to a heteroaryl moiety.

Unless otherwise indicated, the term "alkylheterocycle" or "alkyl-heterocycle" means an alkyl moiety bound to a heterocycle moiety.

Unless otherwise indicated, the term "alkynyl" means a straight chain, branched or cyclic hydrocarbon having from 2 to 20 (*e.g.,* 2 to 20 or 2 to 6) carbon atoms, and including at least one carbon-carbon triple bond. Representative alkynyl moieties include acetylenyl, propynyl, 1-butynyl, 2-butynyl, 1-pentynyl, 2-pentynyl, 3-methyl-1-butynyl, 4-pentynyl, 1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl and 9-decynyl.

Unless otherwise indicated, the term "aryl" means an aromatic ring or an aromatic or partially aromatic ring system composed of carbon and hydrogen atoms. An aryl moiety may comprise multiple rings bound or fused together. Examples of aryl moieties include, but are not limited to, anthracenyl, azulenyl, biphenyl, fluorenyl, indan, indenyl, naphthyl, phenanthrenyl, phenyl, and 1,2,3,4-tetrahydro-naphthalene.

Unless otherwise indicated, the term "arylalkyl" or "aryl-alkyl" means an aryl moiety bound to an alkyl moiety.

Unless otherwise indicated, the terms "halogen" and "halo" encompass fluorine, chlorine, bromine, and iodine.

Unless otherwise indicated, the term "heteroalkyl" refers to an alkyl moiety (linear, branched or cyclic) in which at least one of its carbon atoms has been replaced with a heteroatom (*e.g*., N, O, or S).

Unless otherwise indicated, the term "heteroalkylaryl" or "heteroalkyl-aryl" refers to a heteroalkyl moiety bound to an alkyl moiety.

Unless otherwise indicated, the term "heteroalkylheterocycle" or "heteroalkylheterocycle" refers to a heteroalkyl moiety bound to heterocycle moiety.

Unless otherwise indicated, the term "heteroaryl" means an aryl moiety wherein at least one of its carbon atoms has been replaced with a heteroatom (*e.g*., N, O or S). Examples include, but are not limited to, acridinyl, benzimidazolyl, benzofuranyl, benzoisothiazolyl, benzoisoxazolyl, benzoquinazolinyl, benzothiazolyl, benzoxazolyl, furyl, imidazolyl, indolyl, isothiazolyl, isoxazolyl, oxadiazolyl, oxazolyl, phthalazinyl, pyrazinyl, pyrazolyl, pyridazinyl, pyridyl, pyrimidinyl, pyrimidyl, pyrrolyl, quinazolinyl, quinolinyl, tetrazolyl, thiazolyl, and triazinyl.

Unless otherwise indicated, the term "heteroarylalkyl" or "heteroaryl-alkyl" means a heteroaryl moiety bound to an alkyl moiety.

Unless otherwise indicated, the term "heterocycle" refers to an aromatic, partially aromatic or non-aromatic monocyclic or polycyclic ring or ring system comprised of carbon, hydrogen and at least one heteroatom (*e.g*., N, O or S). A heterocycle may comprise multiple (*i.e.,* two or more) rings fused or bound together. Heterocycles include heteroaryls. Examples include, but are not limited to, benzo[1,3]dioxolyl, 2,3-dihydro-benzo[1,4]dioxinyl, cinnolinyl, furanyl, hydantoinyl, morpholinyl, oxetanyl, oxiranyl, piperazinyl, piperidinyl, pyrrolidinonyl, pyrrolidinyl, tetrahydrofuranyl, tetrahydropyranyl, tetrahydropyridinyl, tetrahydropyrimidinyl, tetrahydrothiophenyl, tetrahydrothiopyranyl and valerolactamyl.

Unless otherwise indicated, the term "heterocyclealkyl" or "heterocycle-alkyl" refers to a heterocycle moiety bound to an alkyl moiety.

Unless otherwise indicated, the term "heterocycloalkyl" refers to a non-aromatic heterocycle.

Unless otherwise indicated, the term "heterocycloalkylalkyl" or "heterocycloalkyl-alkyl" refers to a heterocycloalkyl moiety bound to an alkyl moiety.

Unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

Unless otherwise indicated, the term "pharmaceutically acceptable salts" refers to salts prepared from pharmaceutically acceptable non-toxic acids or bases including inorganic acids and bases and organic acids and bases. Suitable pharmaceutically acceptable base addition salts include, but are not limited to, metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts. Others are well-known in the art. *See, e.g.,* Remington's Pharmaceutical Sciences, 18th ed. (Mack Publishing, Easton PA: 1990) and Remington: The Science and Practice of Pharmacy, 19th ed. (Mack Publishing, Easton PA: 1995).

Unless otherwise indicated, the terms "prevent," "preventing" and "prevention" contemplate an action that occurs before a patient begins to suffer from the specified disease or disorder, which inhibits or reduces the severity of the disease or disorder. In other words, the terms encompass prophylaxis.

Unless otherwise indicated, a "prophylactically effective amount" of a compound is an amount sufficient to prevent a disease or condition, or one or more symptoms associated with the disease or condition, or prevent its recurrence. A "prophylactically effective amount" of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

Unless otherwise indicated, the term "substituted," when used to describe a chemical structure or moiety, refers to a derivative of that structure or moiety wherein one or more of its hydrogen atoms is substituted with a chemical moiety or functional group such as, but not limited to, alcohol, aldehyde, alkoxy, alkanoyloxy, alkoxycarbonyl, alkenyl, alkyl (*e.g*., methyl, ethyl, propyl, cyclopropyl, t-butyl), alkynyl, alkylcarbonyloxy (-OC(O)alkyl), amide (*e.g*. -C(O)NH-alkyl-, -alkylNHC(O)alkyl), amidinyl (*e.g*., -C(NH)NH-alkyl-, -C(NR)NH₂), amine (primary, secondary and tertiary such as alkylamino, arylamino, arylalkylamino), aroyl, aryl, aryloxy, azo, carbamoyl (*e.g.,* -NHC(O)O-alkyl-,-OC(O)NH-alkyl), carbamyl (*e.g.,* CONH₂, CONH-alkyl, CONH-aryl, CONH-arylalkyl), carbonyl, carboxyl, carboxylic acid, carboxylic acid anhydride, carboxylic acid chloride, cyano, ester, epoxide, ether *(e.*g., methoxy, ethoxy), guanidino, halo, haloalkyl (*e.g.,* -CCl₃, -CF₃, -C(CF₃)₃), heteroalkyl, hemiacetal, imine (primary and secondary), isocyanate, isothiocyanate, ketone, nitrile, nitro, oxo, phosphodiester, sulfide, sulfonamido (*e.g*., SO₂NH₂), sulfone, sulfonyl (including alkylsulfonyl, arylsulfonyl and arylalkylsulfonyl), sulfoxide, thiol (*e.g*., sulfhydryl, thioether) and urea (*e.g*., -NHCONH-alkyl-). Particular substituents are alkoxy, alkyl, amino (including alkylamino, dialkylamino), aryl, carboxylic acid, cyano, halo, haloalkyl, heterocycle, and hydroxyl.

Unless otherwise indicated, a "therapeutically effective amount" of a compound is an amount sufficient to provide a therapeutic benefit in the treatment or management of a disease or condition, or to delay or minimize one or more symptoms associated with the disease or condition. A "therapeutically effective amount" of a compound means an amount of therapeutic agent, alone or in combination with other therapies, which provides a therapeutic benefit in the treatment or management of the disease or condition. The term "therapeutically effective amount" can encompass an amount that improves overall therapy, reduces or avoids symptoms or causes of a disease or condition, or enhances the therapeutic efficacy of another therapeutic agent.

Unless otherwise indicated, the terms "treat," "treating" and "treatment" contemplate an action that occurs while a patient is suffering from the specified disease or disorder, which reduces the severity of the disease or disorder, or retards or slows the progression of the disease or disorder.

Unless otherwise indicated, the term "include" has the same meaning as "include, but are not limited to," and the term "includes" has the same meaning as "includes, but is not limited to." Similarly, the term "such as" has the same meaning as the term "such as, but not limited to."

Unless otherwise indicated, one or more adjectives immediately preceding a series of nouns is to be construed as applying to each of the nouns. For example, the phrase "optionally substituted alky, aryl, or heteroaryl" has the same meaning as "optionally substituted alky, optionally substituted aryl, or optionally substituted heteroaryl."

It should be noted that a chemical moiety that forms part of a larger compound may be described herein using a name commonly accorded it when it exists as a single molecule or a name commonly accorded its radical. For example, the terms "pyridine" and "pyridyl" are accorded the same meaning when used to describe a moiety attached to other chemical moieties. Thus, the two phrases "XOH, wherein X is pyridyl" and "XOH, wherein X is pyridine" are accorded the same meaning, and encompass the compounds pyridin-2-ol, pyridin-3-ol, and pyridin-4-ol.

It should also be noted that if the stereochemistry of a structure or a portion of a structure is not indicated with, for example, bold or dashed lines, the structure or the portion of the structure is to be interpreted as encompassing all stereoisomers of it. Moreover, any atom shown in a drawing with unsatisfied valences is assumed to be attached to enough hydrogen atoms to satisfy the valences. In addition, chemical bonds depicted with one solid line parallel to one dashed line encompass both single and double (*e.g*., aromatic) bonds, if valences permit.

### 5.2. Compounds

This invention encompasses compounds: 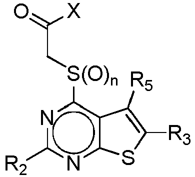 or a pharmaceutically acceptable salt thereof, wherein: X is OR_{1B} or N(R_{1B})₂; each R_{1B} is independently hydrogen or optionally substituted alkyl, aryl, heteroalkyl, or heterocycle; R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle; each R_{2A} is independently hydrogen or optionally substituted alkyl, aryl, heteroalkyl, or heterocycle; R₃ is hydrogen, halo, cyano, -
CO₂R_{3A}, -C(O)N(R_{3A})₂, -SR_{3A}, -OR_{3A}, -N(R_{3A})₂, or optionally substituted alkyl, aryl, heteroalkyl, or heterocycle; each R_{3A} is independently hydrogen or optionally substituted alkyl, aryl, heteroalkyl, or heterocycle; R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle; each R_{5A} is independently hydrogen or optionally substituted alkyl, aryl, heteroalkyl, or heterocycle; and n is 1 or 2; wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

The invention also encompasses compounds of the formula: 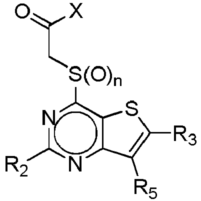 or a pharmaceutically acceptable salt thereof, wherein: X is OR_{1B} or N(R_{1B})₂; each R_{1B} is independently hydrogen or optionally substituted alkyl, aryl, heteroalkyl, or heterocycle; R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle; each R_{2A} is independently hydrogen or optionally substituted alkyl, aryl, heteroalkyl, or heterocycle; R₃ is hydrogen, halo, cyano, -
CO₂R_{3A}, -C(O)N(R_{3A})₂, -SR_{3A}, -OR_{3A}, -N(R_{3A})₂, or optionally substituted alkyl, aryl, heteroalkyl, or heterocycle; each R_{3A} is independently hydrogen or optionally substituted alkyl, aryl, heteroalkyl, or heterocycle; R₄ is hydrogen, halo, or optionally substituted alkyl, aryl, heteroalkyl, or heterocycle; R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle; each R_{5A} is independently hydrogen or optionally substituted alkyl, aryl, heteroalkyl, or heterocycle; and n is 1 or 2; wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

In some compounds, R₂ is hydrogen, halo, or lower alkyl. In particular compounds, R₂ is hydrogen.

In some, R₃ is cyano, halo, hydroxyl, or optionally substituted alkyl.

In some, R₄ is hydrogen.

In some, R₅ is cyano, halo, hydroxyl, or optionally substituted alkyl.

In particular compounds of this formula, X is OR_{1B}.

In some, R_{1B} is hydrogen or optionally substituted alkyl or aryl.

In some, R₂ is hydrogen or optionally substituted alkyl.

In some, R₃ is alkyl or halo.

In some, R₃ is chloro.

In some, R₅ is alkyl or halo.

In some, R₅ is C₁₋₄-alkyl.

A particular embodiment is such that the compound is not 2-((6,7-diphenylfuro[3,2-d]pyrimidin-4-yl)thio)-1-morpholinoethanone. One embodiment is such that R₃ and R₅ are not both phenyl.

Particular compounds have an IC₅₀ of less than about 0.1, 0.05, or 0.025 µM as determined by the binding assay described herein. Particular compounds have an EC₅₀ of less than about 5, 2.5, or 1 µM as determined by the reporter assay described herein.

Compounds of the invention (*i.e.,* compounds disclosed herein) can be prepared by methods disclosed herein as well as by methods known in the art. *See, e.g.,* U.S. patent no. 6,579,882 to Stewart et al.; EP patent no. 0447891 to Wiesenfeldt et al.

### 5.3. Methods of Treatment

This invention relates to a method of stimulating endocortical bone formation in a patient, which comprises administering to a patient in need thereof an effective amount of a compound of the invention. It also relates to a method of increasing cortical bone thickness, comprising administering to a patient in need thereof an effective amount of a compound of the invention.

This invention relates to a method of treating, managing, or preventing a disease or disorder associated with bone loss, which comprises administering to a patient in need thereof a therapeutically or prophylactically effective amount of a compound of the invention. Examples of diseases and disorders include osteoporosis (*e.g*., postmenopausal osteoporosis, steroid- or glucocorticoid-induced osteoporosis), osteopenia, and Paget's disease.

Also related to by the invention is a method of treating, managing, or preventing bone fractures, which comprises administering to a patient in need thereof a therapeutically or prophylactically effective amount of a compound of the invention. Particular bone fractures are associated with metastatic bone disease, *i.e.,* cancer that has metastasized to bone. Examples of cancers that can metastasize to bone include prostate, breast, lung, thyroid, and kidney cancer.

This invention also relates to a method of treating, managing, or preventing bone loss associated with, or caused by, a disease or disorder, which comprises administering to a patient in need thereof a therapeutically or prophylactically effective amount of a compound of the invention. Examples of diseases and disorders include celiac disease, Crohns Disease, Cushing's syndrome, hyperparathyroidism, inflammatory bowel disease, and ulcerative colitis.

Examples of patients that may benefit from methods of this invention include men and women aged 55 years or older, post-menopausal women, and patients suffering from renal insufficiency.

Compounds of the invention can be administered in combination (*e.g*., at the same or at different times) with other drugs known to be useful in the treatment, management, or prevention of diseases or conditions affecting the bone. Examples include: androgen receptor modulators; bisphosphonates; calcitonin; calcium sensing receptor antagonists; cathepsin K inhibitors; estrogen and estrogen receptor modulators; integrin binders, antibodies, and receptor antagonists; parathyroid hormone (PTH) and analogues and mimics thereof; and Vitamin D and synthetic Vitamin D analogues.

Examples of androgen receptor modulators include finasteride and other 5α-reductase inhibitors, nilutamide, flutamide, bicalutamide, liarozole, and abiraterone acetate.

Examples of bisphosphonates include alendronate, cimadronate, clodronate, etidronate, ibandronate, incadronate, minodronate, neridronate, olpadronate, pamidronate, piridronate, risedronate, tiludronate, and zolendronate, and pharmaceutically acceptable salts and esters thereof.

Examples of cathepsin K inhibitors include VEL-0230, AAE581 (balicatib), MV061194, SB-462795 (relacatib), MK-0822 (odanacatib), and MK-1256.

Examples of estrogen and estrogen receptor modulators include naturally occurring estrogens (*e.g.,* 7-estradiol, estrone, and estriol), conjugated estrogens (*e.g.,* conjugated equine estrogens), oral contraceptives, sulfated estrogens, progestogen, estradiol, droloxifene, raloxifene, lasofoxifene, TSE-424, tamoxifen, idoxifene, LY353381, LY117081, toremifene, fulvestrant, 4-[7-(2,2-dimethyl-1-oxopropoxy-4-methyl-2-[4-[2-(1-piperidinyl)ethoxy]phenyl]-2H-1-benzopyran-3-yl]-phenyl-2,2-dimethylpropanoate, 4,4'-dihydroxybenzophenone-2,4-dinitrophenyl-hydrazone, and SH646.

Examples of integrin binders, antibodies, and receptor antagonists include vitaxin (MEDI-522), cilengitide and L-000845704.

### 5.4. Pharmaceutical Formulations

This invention encompasses pharmaceutical compositions comprising one or more compounds of the invention, and optionally one or more other drugs, such as those described above.

Certain pharmaceutical compositions are single unit dosage forms suitable for oral, mucosal (*e.g.,* nasal, sublingual, vaginal, buccal, or rectal), parenteral (*e.g.,* subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g.,* nasal sprays or inhalers); gels; liquid dosage forms suitable for oral or mucosal administration to a patient, including suspensions (*e.g.,* aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g.,* crystalline or amorphous solids) that can be reconstituted to provide liquid dosage forms suitable for parenteral administration to a patient.

The formulation should suit the mode of administration. For example, oral administration requires enteric coatings to protect the compounds of this invention from degradation within the gastrointestinal tract. Similarly, a formulation may contain ingredients that facilitate delivery of the active ingredient(s) to the site of action. For example, compounds may be administered in liposomal formulations, in order to protect them from degradative enzymes, facilitate transport in circulatory system, and effect delivery across cell membranes to intracellular sites.

The composition, shape, and type of a dosage form will vary depending on its use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms encompassed by this invention will vary from one another will be readily apparent to those skilled in the art. *See, e.g.,* Remington's Pharmaceutical Sciences, 18th ed. (Mack Publishing, Easton PA: 1990).

Pharmaceutical compositions of this invention are preferably administered orally. Discrete dosage forms suitable for oral administration include tablets (*e.g.,* chewable tablets), caplets, capsules, and liquids (*e.g.,* flavored syrups). Such dosage forms contain predetermined amounts of active ingredients, and may be prepared by methods of pharmacy well known to those skilled in the *art. See, e.g.,* Remington's Pharmaceutical Sciences, 18th ed. (Mack Publishing, Easton PA: 1990).

Typical oral dosage forms are prepared by combining the active ingredient(s) in an intimate admixture with at least one excipient according to conventional pharmaceutical compounding techniques. Excipients can take a wide variety of forms depending on the form of preparation desired for administration.

Because of their ease of administration, tablets and capsules represent the most advantageous oral dosage unit forms. If desired, tablets can be coated by standard aqueous or nonaqueous techniques. Such dosage forms can be prepared by conventional methods of pharmacy. In general, pharmaceutical compositions and dosage forms are prepared by uniformly and intimately admixing the active ingredients with liquid carriers, finely divided solid carriers, or both, and then shaping the product into the desired presentation if necessary. Disintegrants may be incorporated in solid dosage forms to facility rapid dissolution. Lubricants may also be incorporated to facilitate the manufacture of dosage forms (*e.g.,* tablets).

### 6. EXAMPLES

### 6.1. Knock-out Mouse

Mice homozygous for a genetically engineered mutation in the murine ortholog of the human NOTUM gene were generated using corresponding mutated embryonic stem (ES) cell clones from the OMNIBANK collection of mutated murine ES cell clones (see generally, U.S. Patent No. 6,080,576). In brief, ES cell clones containing a mutagenic viral insertion into the murine NOTUM locus were microinjected into blastocysts which were in turn implanted into pseudopregnant female hosts and carried to term. The resulting chimeric offspring were subsequently bred to C57 black 6 female mice and the offspring checked for the germline transmission of the knocked-out NOTUM allele. Animals heterozygous for the mutated NOTUM allele were subsequently bred to produce offspring that were homozygous for the mutated NOTUM allele, heterozygous for the mutated NOTUM allele, or wild type offspring at an approximate ratio of 1:2:1.

Mice homozygous (-/-) for the disruption of the NOTUM gene were studied in conjunction with mice heterozygous (+/-) for the disruption of the NOTUM gene and wild-type (+/+) litter mates. During this analysis, the mice were subject to a medical work-up using an integrated suite of medical diagnostic procedures designed to assess the function of the major organ systems in a mammalian subject. By studying the homozygous (-/-) "knockout" mice in the described numbers and in conjunction with heterozygous (+/-) and wild-type (+/+) litter mates, more reliable and repeatable data were obtained.

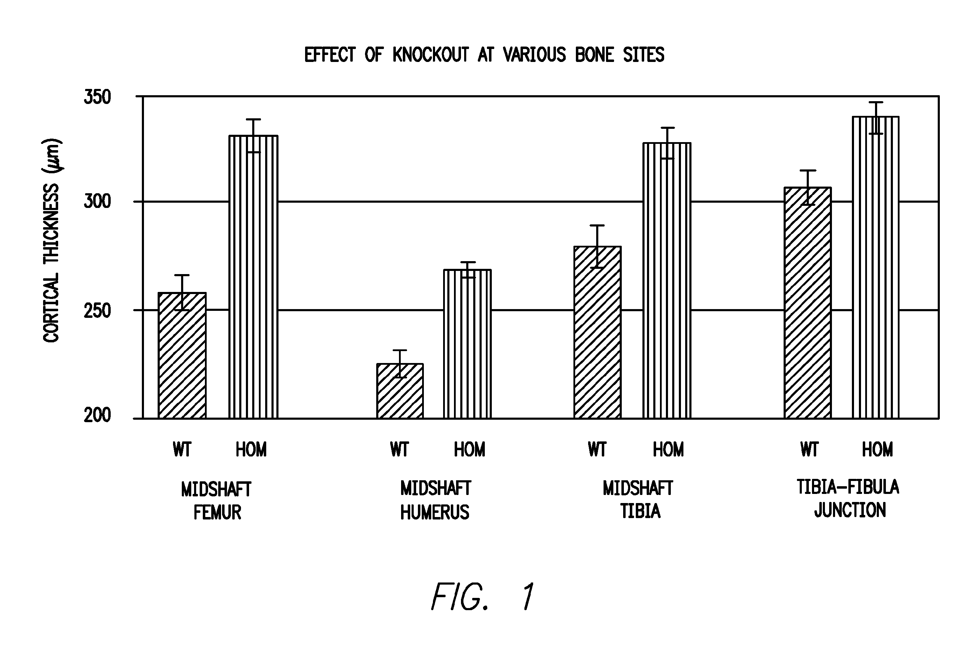

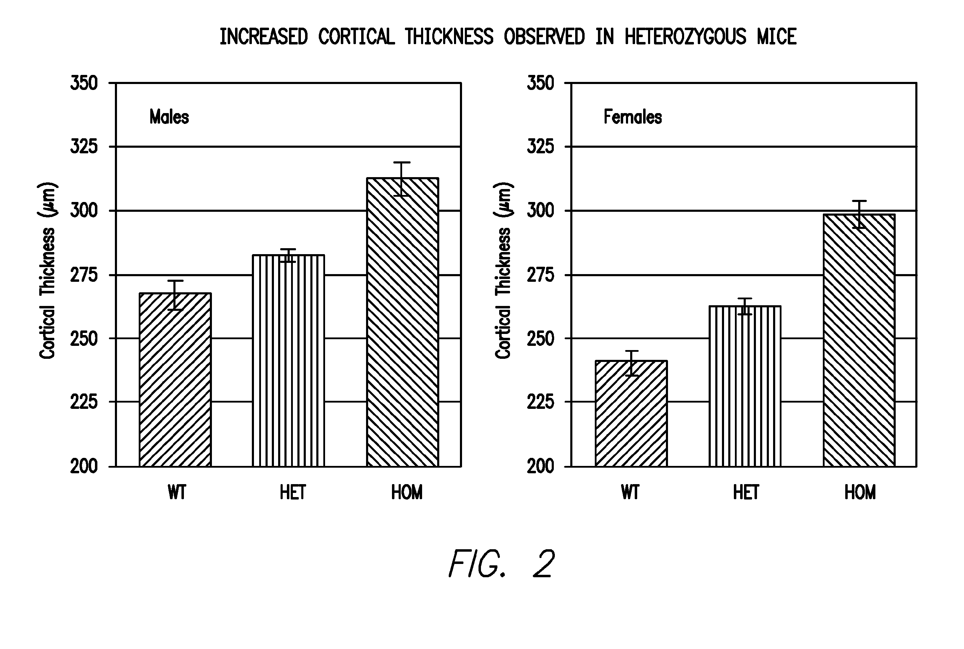

As shown in Figure 1, male mice having homozygous disruption of the NOTUM gene ("homs") exhibited greater cortical thicknesses at various bone sites, compared to their wildtype littermates at 16 weeks of age (number of mice N = 10 for both groups). These differences, which were measured by microCT (Scanco µCT40), were: 28% (p < 0.001) at midshaft femur; 19% (p < 0.001) at midshaft humerous; 17% (p < 0.001) at midshaft tibia; and 11% (p < 0.001) at tibia-fibula junction. As shown in Figure 2, at 16 weeks of age, the midshaft femur cortical bone thickness of mice heterozygous for the NOTUM mutation ("hets") was also greater than that of their wildtype littermates: male hets (N = 50) exhibited a 6% (p = 0.007) increase compared to their wildtype littermates (N = 23); and female hets (N = 57) exhibited a 9% (p < 0.001) increase compared to their wildtype littermates (N = 22).

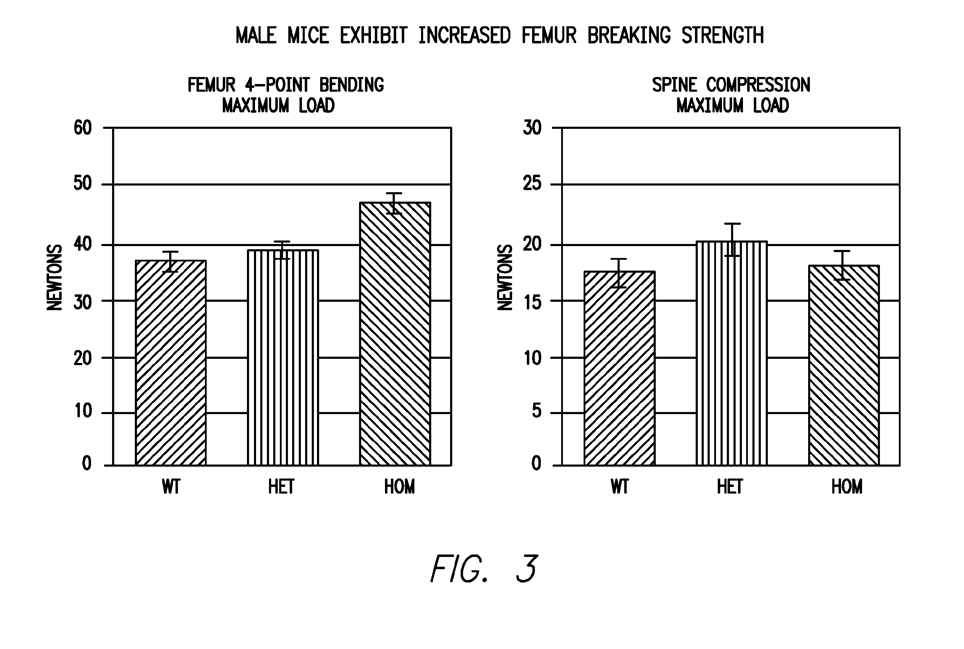

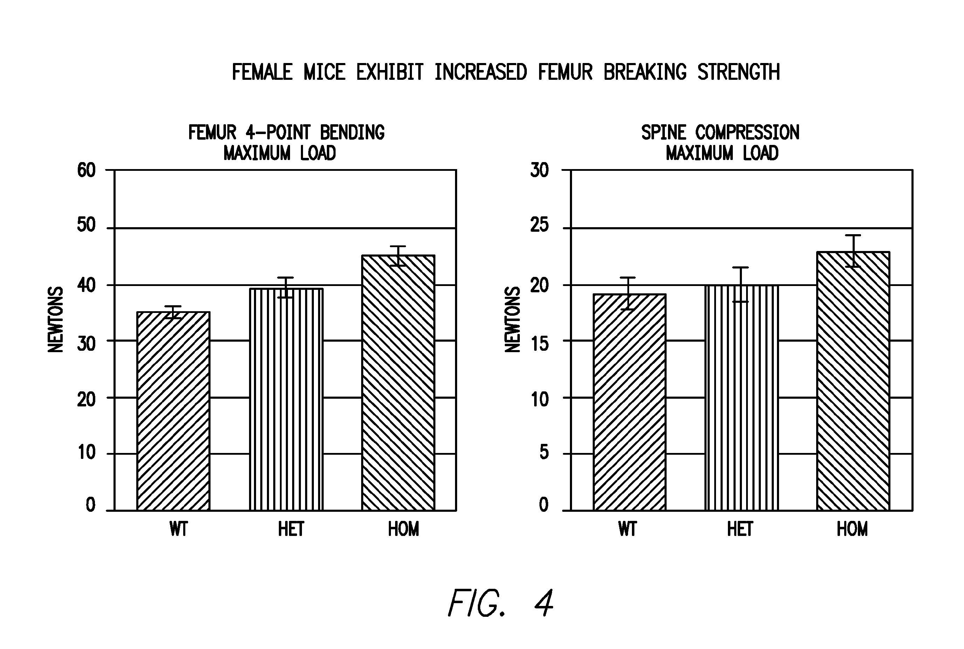

Practical manifestations of the observed redistribution of bone formation in NOTUM animals is reflected in Figures 3 and 4, which show results of femur breaking strength tests (performed by SkeleTech, now Ricerca Biosciences) using a standard 4-point bending test. As shown in Figure 3, which provides results obtained for male mice at 16 weeks of age, hets (N = 20) exhibited a 5% ( p = 0.54) increase in femur breaking strength compared to their wildtype littermates (N = 23), whereas homs (N = 17) exhibited a 28% (p < 0.001) increase. On the other hand, spine compression tests of both NOTUM homs and hets did not show a significant reduction in maximum spine compression loads as compared to wildtype controls. Similar results were obtained for female mice at 16 weeks of age. As shown in Figure 4, hets (N = 20) exhibited a 12% ( p = 0.04) increase in femur breaking strength compared to their wildtype littermates (N = 21), whereas homs (N = 18) exhibited a 28% (p < 0.001) increase. Analyzation of these and other data revealed a strong correlation between cortical thickness and femur breaking strength.

### 6.2. Reporter Assay

Compounds' EC₅₀ values were determined using this assay, which utilized conditioned media that was prepared as follows. Plasmid containing human notum pectinacetyltransferase in pcDNA3.1(+) vector was transfected into HEK293 cells and clones were selecting by growing in presence of 400 ug/mL of G418. The clone containing highest expression of human notum pectinacetyltransferase in the conditioned media was maintained for all future activity assays. L cells overexpressing and secreting Wnt3a into the conditioned media were purchased from ATCC.

The assay protocol was as follows. Approximately 5 million CellSensor®LEF/TCF-bla Freestyle™ 293F cells were grown to near confluency in 15-cm plates. The cell growth medium consisted of DMEM with 10% Dialyzed FBS, 5 µg/ml Blasticidin (Invitrogen, R210-01), 0.1 mM NEAA, 25 mM HEPES and 1×GPS. Cells were then trypsinized by first rinsing with PBS, followed by addition of 5 mL of trypsin and incubation of plates at room temperature for two minutes. A total of 10 mL of assay media (Opti-MEM, plus 0.5% dialyzed FBS, 0.1 mM NEAA, 1mM sodium pyruvate, 10 mM HEPES, 1x GPS) was then added per 15 cm plate. Cells were counted and suspended at 0.75 million cells per mL. Cells were seeded into Biocoat 384-well plates (Fisher, Catalogue #356663) at a density of 15000 cells per 20 µL per well. After incubation of cells at 37°C for 3 hours, 10 µL of 30 mM LiCl in assay medium was added per well, followed by incubation at 37°C for another 3 hours. Meanwhile, compounds were acoustically pinged into Greiner 384-well plates (catalog # 781076) using an ECHO, followed by addition of 10 µL per well of Wnt3a conditioned media and 10 µL per well of notum pectinacetylesterase-conditioned media. Ten µL of the Wnt3a/notum pentinacetyltransferase mixture was then transferred from Greiner plates to each well of the 384-well plates containing the CellSensor cells. After incubation of cells overnight at 37°C, reactions were developed by addition of 5 µL of 1xCCF4 (Invitrogen, Catalogue number K1085) to each well, covering the entire 384-well plate, and gentle rocking in the dark at room temperature for 3 hours. Plates were then read on an Envision Plate Reader using an excitation wavelength of 400 nm and emission wavelengths of 460 nm and 535 nm.

### 6.3. Binding Assay

Compounds' IC₅₀ values were determined using this assay, which utilized trisodium 8-octanoyloxypyrene-1,3,6-trisulfonate (OPTS), a water soluble enzyme substrate for fluorimetric assays of esterases and lipases. Plasmid containing human notum pectinacetyltransferase in pcDNA3.1(+) vector was transfected into HEK293 cells and clones were selecting by growing in presence of 400 ug/mL of G418. Condition media from these cells was used for the assay.

An ECHO was used to acoustically dispense 75 nL of compounds into dry Greiner 384-well plates (catalog # 781076), followed by addition of 10 uL of 50 mM Tris/HCl (pH 6.8) to every well of these 384-well assay plates. Conditioned media containing human notum pectinacetyltransferase was diluted 75x with Assay Buffer (50 mM Tris, pH 6.8, 5 mM CaCl₂, 0.5 mM MgCl₂), and 25 µL of this "Enzyme Mix" was added to each well followed by a 10 minute pre-incubation. Enzyme reactions were initiated by addition of 15 µL OPTS substrate (Sigma, catalog # 74875) to a final concentration of 5 µM and reaction times were for 10 minutes at room temperature. All plates were read on an Envision Plate Reader with an excitation wavelength of 485nm and emission wavelength of 535 nm.

### 6.4. General Synthetic Method A: Preparation of 3-chloro-2-methylthieno[2,3-b]pyridine-4-ol (2); 3-bromo-2-methylthieno[2,3-b]pyridine-4-ol (3)

This general approach to the synthesis of compounds encompassed by the invention is illustrated by the specific example described below:

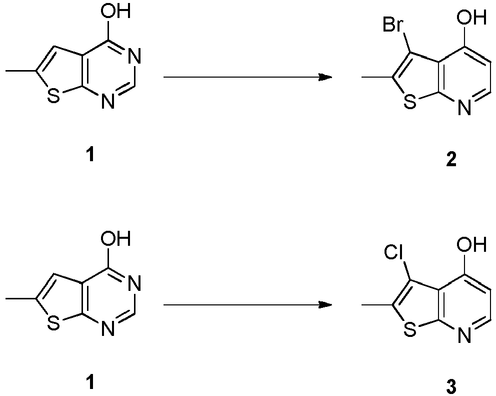

In a 100 mL round bottom flask 6-methylthieno[2,3-d]pyrimidin-4-ol **1** (1g, 6.2 mmol), was taken up in acetic acid (12 mL) and stirred at room temperature. To this bromine (0.3 mL, 6.2 mmol) was added and the reaction mixture was stirred at room temperature for overnight. The reaction was then quenched with ice and stirred until ice was melted. Product was then filtered off washed with water and dried to obtain (1g, 66% yield) 3-bromo-2-methylthieno[2,3-b]pyridine-4-ol **2** as a dry product.

In a 3 L round bottom flask 6-methylthieno[2,3-d]pyrimidin-4-ol **1** (50 g, 310 mmol), was taken up in acetic acid (525 mL) and NCS (37.5 g, 372 mmol) was added. The reaction was heated at 55°C for 8 hours. The reaction was then cooled to room temperature and quenched with water and extracted the compound with methylene chloride (2 x 250 mL). The combined organic layers were washed with brine and dried over MgSO₄, filtered and concentrated, yielding 3-chloro-2-methylthieno[2,3-b]pyridine-4-ol as a solid **3** in (42.15 g, 70% yield).

### 6.5. General Synthetic Method B: Preparation of 2-(5-chloro-6-methylthieno[2,3-d]pyrimidin-4-ylthio)acetic acid (6)

This general approach to the synthesis of compounds encompassed by the invention is illustrated by the specific example described below:

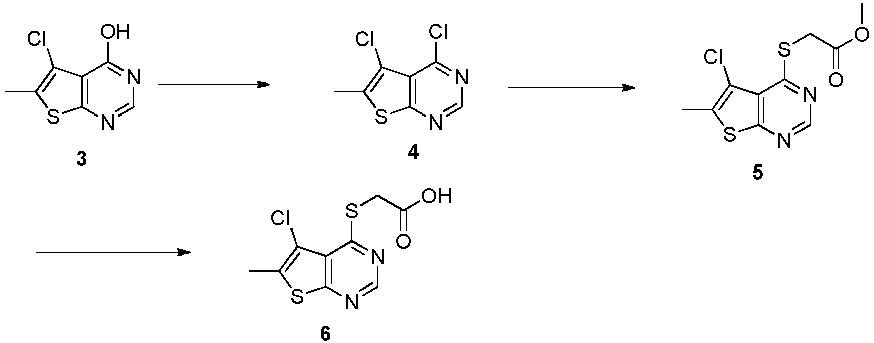

**Synthesis of 4:** 3-chloro-2-methylthieno[2,3-b]pyridine-4-ol 3 (40 g, 2000 mmol, 1 eq.) was suspended in POCl₃ (368 mL, 4000 mmol, 20 eq.) and heated to 90°C for 4 hours. After cooling the reaction was concentrated and the residue cooled to 0°C. Ice cold water was then added slowly to ensure complete consumption of POCl₃. The black solution was thrice extracted with ethyl acetate. The combined organic extracts were twice washed with saturated aqueous NaHCO₃, once with brine, dried over MgSO₄, filtered and concentrated, yielding light brown solid 4,5-dichloro-6-methylthieno[2,3-d]pyrimidine **4** (41.6 g, 93% yield), which was immediately used after complete drying.

**Synthesis of 5:** 4,5-dichloro-6-methylthieno[2,3-d]pyrimidine **4** (40 g, 182.2 mmol, 1 eq.) was suspended in methanol (500 mL) and cooled to 0°C. Methyl 2-mercaptoacetate (27 mL, 191.5 mmol, 1.05 eq.) was added slowly, followed by the slow addition of TEA (53 mL, 382.6 mmol, 2.1 eq.). After stirring for 90 minutes, the reaction was concentrated. The residue was taken up in ethyl acetate and filtered through a silica gel plug to remove salts and some color. The filtrate was concentrated and purified using silica gel chromatography with 0-15% ethyl acetate in hexanes, yielding methyl 2-(5-chloro-6-methylthieno[2,3-d]pyrimidin-4-ylthio)acetate **5** (47.30 g, 91% yield) as an off white solid.

**Synthesis of 6:** 2-(5-chloro-6-methylthieno[2,3-d]pyrimidin-4-ylthio)acetate 5 (45 g, 150 mmol, 1 eq.) was taken up in THF (350 mL) and 1 N aqueous NaOH (300 mL, 300 mmol, 2 eq.) was added, with efficient stirring. After 40 minutes, the reaction was concentrated and the residue taken up in water, cooled to 0°C and acidified to low pH with 1 N HCl The precipitate was collected via vacuum filtration, washed with water. Drying it under vacuum for overnight, yielded pure 2-(5-chloro-6-methylthieno[2,3-d]pyrimidin-4-ylthio)acetic acid **6** (40.5 g, 94% yield) as a white solid. **MS** *m*/*z* C₉H₇ClN₂O₂S₂ [M+1]⁺ = 275 and [M+1]⁺³ = 277. **¹H NMR** (400 MHz, METHANOL-*d*₄) δ ppm 8.66 (s, 1 H), 4.05 (s, 2 H), 2.54 (s, 3 H). **¹³C NMR** (101 MHz, DMSO-*d*₆) δ ppm 170.04 (s), 162.55 (s, 1C), 162.48 (s, 1C), 152.55 (s, 1C), 135.03 (s, 1C), 125.03 (s, 1C), 114.36 (s, 1C), 32.17 (s, 1C), 14.05 (s, 1C).

### 6.6. General Synthetic Method C: Preparation of 2-(6-bromo-5-methylthieno[2,3-d]pyrimidin-4-ylthio)acetic acid (10)

This general approach to the synthesis of compounds encompassed by the invention is illustrated by the specific example described below: 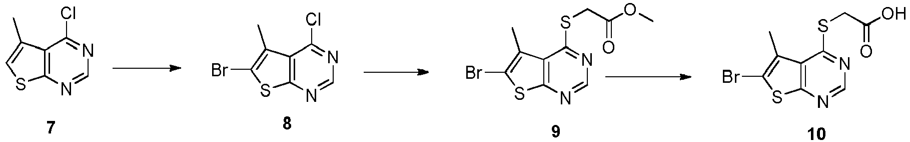 **Synthesis of 8:** 4-Chloro-5-methylthiene[2,3-d]pyrimidine **7** (1g, 5.4mmol), prepared according to General Synthetic Method **B**, was taken up in acetic acid and stirred at room temperature. To this reaction mixture, bromine (1eq = 275 µL, 5.4 mmol) was added and the reaction mixture was stirred for overnight. The reaction was then quenched with ice and stirred until ice melted. Product was then filtered off washed with water and dried to obtain 1g of 6-bromo-4-chloro-5-methylthieno[2,3-d]pyrimidine **8** (786 mg, 55% yield) as a pale yellow solid.

Compounds **9** and **10** were prepared according to General Synthetic Method **B**.

### 6.7. General Synthetic Method D: Preparation of methyl 2-(5-cyclopropyl-6-methylthieno[2,3-d]pyrimidin-4-ylthio)acetate (12)

This general approach to the synthesis of compounds encompassed by the invention is illustrated by the specific example described below:

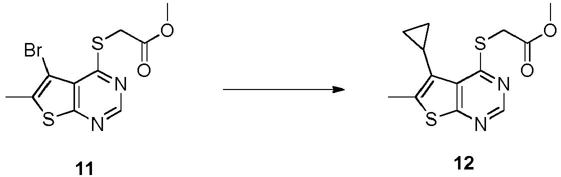

In a 100 mL round bottom flask was charged with methyl 2-(5-bromo-6-methylthieno[2,3-d]pyrimidin-4-ylthio)acetate **11** (0.100 g, 0.3 mmol, 1 eq), cyclopropyl boronic acid (51 mgs, 0.6 mmol) and trycyclohexylphosphine (8.4 mgs, 0.03 mmol) and K₃PO₄ (212 mgs, 1.05 mmol) were taken in toluene, water mixture (4 mL, 1:1). This mixture was stirred at room temperature under N₂ atmosphere. To this stirred solution diacetoxy palladium (4 mgs, 0.015 mmol) was added. Then the reaction mixture was heated at 100°C for 8 hrs. The reaction was quenched with the addition of 1 N HC! and the aqueous portion extracted thrice with EtOAc. The combined organic portions were dried over MgSO₄, filtered and concentrated to yield methyl 2-(5-cyclopropyl-6-methylthieno[2.3-d]pyrimidin-4-ylthio)acetate (40 mgs, 45% yield) **12** as a light yellow solid.

### 6.8. General Synthetic Method E: Preparation of methyl 2-(6-ethyl-5-methylthieno[2,3-d]pyrimidin-4-ylthio)acetate (13)

This general approach to the synthesis of compounds encompassed by the invention is illustrated by the specific example described below:

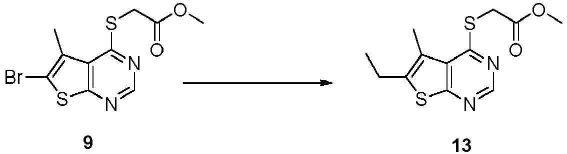

In a 100 mL round bottom flask charged with methyl 2-(6-bromo-5-methylthieno[2,3-d]pyrimidin-4-ylthio)acetate **9** (50mg, 0.15mmol), prepared according to General Synthetic Method C, and diethylzinc (1.1M in toluene, 204 µL, 0.23mmol), Pd(dba)₃ (4 mg, 0.0075 mmol), and 2-dicyclohexylphosphine-2',6'-isopropoxydiphenyl (13 mg, 0.03 mmol) were taken up in dry DMF (2.5mL) in a microwave vessel and heated under microwave irradiation at 160°C for 10 minutes. After it was cooled to room temperature it was filtered through celite and concentrated. This was taken up in EtOAc and washed with water. The organic layer was washed with brine and dried over magnesium sulfate and concentrated to afford methyl 2-(6-ethyl-5-methylthieno[2,3-d]pyrimidin-4-ylthio)acetate **13** (17 mgs, 40% yield).

### 6.9. General Synthetic Method F: Preparation of 2-(5-(dimethylamino)-6-methylthieno[2,3-d]pyrimidin-4-ylthio)acetic acid (14)

This general approach to the synthesis of compounds encompassed by the invention is illustrated by the specific example described below:

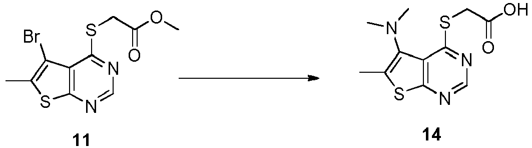

Methyl 2-(5-bromo-6-methylthieno[2.3-d]pyrimidin-4-ylthio)acetate **11** (100mg, 0.3 mmol), prepared using General Synthetic Method C, was taken up in 1mL (excess, as solvent) of dimethyl amine 40% in water. A catalytic amount of copper oxide was then added and the reaction heated under microwave irradiation at 140°C for 15 min. Reaction stripped down then purified by preparative HPLC (aqueous ammonium acetate / acetonitrile). Isolated **14** as a white solid (60 mgs, 71% yield).

### 6.10. General Synthetic Method G: Preparation of 2-(5,6-dimethylthieno[2,3-d]pyrimidin-4-ylthio)-N-(2-methoxyethyl)acetamide (17)

This general approach to the synthesis of compounds encompassed by the invention is illustrated by the specific example described below:

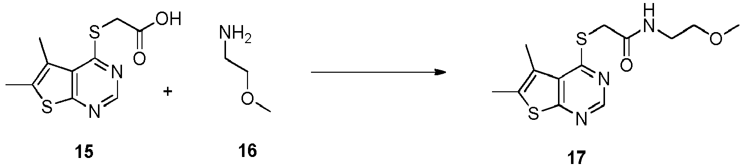

2-(5,6-dimethylthieno[2,3-d]pyrimidin-4-ylthio)acetic acid **15** (0.050 g, 0.2 mmol, 1 eq) ), which is commercially available and can also be prepared using General Synthetic Method **B**, was taken up in DMF (2.2 mL). To this mixture 2-methoxyethanamine **16** (0.019 g, 0.24 mmol, 1.2 eq), HATU (0.076 g, 0.2 mmol, 1 eq) and N,N-diisopropyl ethylamine (0.08 mL, 0.44 mmol, 2.2 eq) was added and stirred the reaction mixture for 8 hrs at room temperature. Water was added to quench and the aqueous portion was extracted thrice with EtOAc. The combined organic layers were dried over MgSO₄, filtered and concentrated. The residue was purified by preparative HPLC using neutral conditions yielding 2-(5,6-dimethylthieno[2,3-d]pyrimidin-4-ylthio)-N-(2-methoxyethyl)acetamide **17** (56 mg, 90% yield) as an off white solid.

### 6.11. General Synthetic Method H: Preparation of Isopropyl 2-(5,6-dimethylthieno[2,3-d]pyrimidin-4-ylthio)acetate (19)

This general approach to the synthesis of compounds encompassed by the invention is illustrated by the specific example described below:

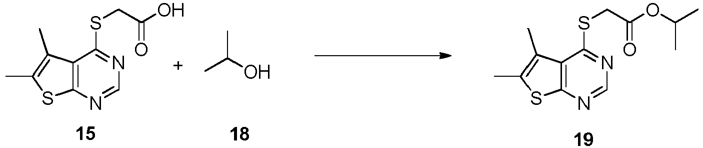

Compound **15** (100 mg, 0.4 mmol), which is commercially available and can also be prepared using General Synthetic Method **B**, was dissolved in dry DCM and thionyl chloride (56 uL, 0.8 mmol) added and stirred at room temperature for 1 hour. Then excess propane-2-ol **18** was added and stirred at room temp another 1 hour. Then, the reaction mixture was concentrated and purified using neutral phase preparative HPLC (aqueous ammonium acetate / acetonitrile) to give **19** as a solid (100 mg, 85% yield).

### 6.12. General Synthetic Method I: Preparation of 2-(5-chloro-6-isopropylthieno[2,3-d]pyrimidin-4-ylthio)acetic acid (24)

This general approach to the synthesis of compounds encompassed by the invention is illustrated by the specific example described below:

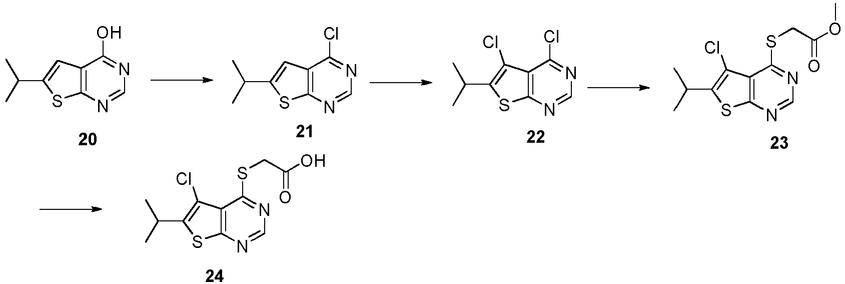

**Synthesis of 21:** Pyrimidone **20** (10 g, 51.4 mmol, 1 eq.), which is commercially available and can also be prepared by General Synthetic Method **D,** was suspended in POCl₃ (95 mL, 1028 mmol, 20 eq.) and heated to 90°C for 4 hours. After cooling the reaction was concentrated and the residue cooled to 0°C. Ice cold water was then added slowly to ensure complete consumption of POCl₃. The black solution was thrice extracted with ethyl acetate. The combined organic extracts were twice washed with saturated aqueous NaHCO₃, once with brine, dried over MgSO₄, filtered and concentrated, yielding light brown solid 4-chloro-6-isopropylthieno[2,3-d]pyrimidine **21** i(10.16 g, 93% yield), which was immediately used after complete drying.

**Synthesis of 22:** Solid 4-chloro-6-isopropylthieno[2,3-d]pyrimidine **21** (9.2 g, 43.2 mmol, 1 eq.) was taken up in acetic acid (75 mL) and NCS (8.65 g, 86.5 mmol, 2 eq.) was added. The reaction was heated at 55°C for 6 hours. The reaction was cooled and quenched with water and extracted the compound with methylene chloride (2 x 50 mL). The combined organic layers were washed with brine and dried over MgSO₄, filtered and concentrated, yielding 4,5-dichloro-6-isopropylthieno[2,3-d]pyrimidine as a solid **22** (6.2 g, 58% yield).

**Synthesis of 23:** 4,5-dichloro-6-isopropylthieno[2,3-d]pyrimidine **22** (11.6 g, 47 mmol, 1 eq.) was suspended in methanol (125 mL) and cooled to 0°C. Methyl 2-mercaptoacetate (6.86 mL, 49.35 mmol, 1.05 eq.) was added slowly, followed by the slow addition of TEA (13.7 mL, 98.7 mmol, 2.1 eq.). After stirring for 90 minutes, the reaction was concentrated. The residue was taken up in ethyl acetate and filtered through a silica gel plug to remove salts and some color. The filtrate was concentrated and purified using silica gel chromatography with 0-15 % ethyl acetate in hexanes, yielding methyl 2-(5-chloro-6-isopropylthieno[2,3-d]pyrimidin-4-ylthio)acetate **23** (13.6 g, 92% yield) as an off white solid.

**Synthesis of 24:** Compoundr **23** (5.0 g, 15.8 mmol, 1 eq.) was taken up in THF (50 mL) and 1 N aqueous NaOH (32 mL, 32 mmol, 2 eq.) was added, with efficient stirring. After 40 minutes, the reaction was concentrated and the residue taken up in water, cooled to 0 °C and acidified to low pH with 1 N HCl. The precipitate was collected via vacuum filtration, washed with water. Drying it under vacuum for overnight, yielded pure 2-(5-chloro-6-isopropylthieno[2,3-d]pyrimidin-4-ylthio)acetic acid **24** as a white solid (4.5 g, 94% yield). **MS** *m*/*z* C₁₁H₁₁ClN₂O₂S₂ [M+1]⁺ = 303 and [M+1]⁺³ = 305. ¹H NMR (400 MHz, DMSO-*d*₆) δ ppm 12.83 (s, 1 H), 8.81 (s, 1 H), 4.12 (s, 2 H), 3.52 - 3.76 (m, 1 H), 1.32 (d, *J*=7.03 Hz, 6 H). **¹³C NMR** (101 MHz, DMSO-d₆) δ ppm 170.0 (s, 1C), 162.8 (s, 1C), 162.2 (s, 1C), 152.6 (s, 1C), 146.9 (s, 1C), 125.1 (s, 1C), 112.3 (s, 1C), 32.2 (s, 1C), 28.6 (s, 1C), 23.4 (s, 1C), 23.1 (s, 1C).

### 6.13. General Synthetic Method J: Preparation of 2-(6-chloro-7-cyclopropylthieno[2,3-d]pyrimidin-4-ylthio)acetic acid (31)

This general approach to the synthesis of compounds encompassed by the invention is illustrated by the specific example described below:

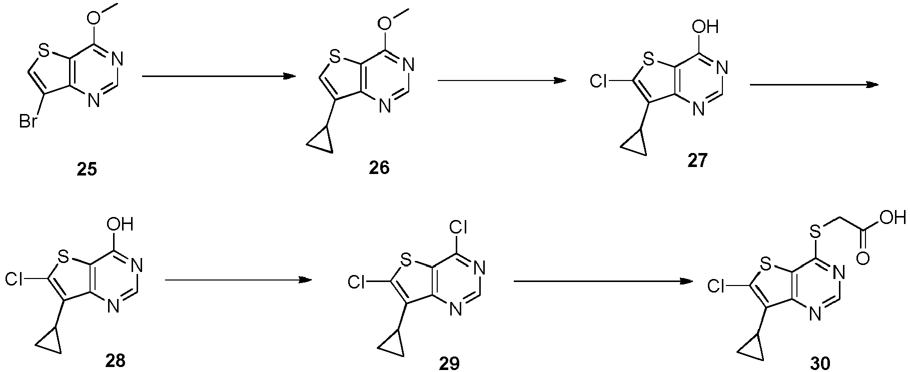

**Synthesis of 26:** In a 500 mL round bottom flask charged with 7-bromo-4-methoxythieno[3,2-d]pyrimidine **25** (4 g, 16.32 mmol, 1 eq) and cyclopropylboronic acid (2.81 g, 32.6 mmol, 2 eq) and potassium carbonate (6.76 g, 49 mmol, 3 eq) and the catalyst, PdCl₂(Ph₃P)₂ (575 mg, 0.816 mmol, 0.05 eq) were taken in a toluene (60 mL and H₂O (16 mL) mixture. Purged the reaction mixture with N₂ strongly for five minutes and heated the reaction mixture at 100°C for 6 hours. After it is cooled to room temperature the reaction mixture was filtered and split both layers. The aqueous layer was washed with toluene and combined organic solvents were concentrated. The crude material was purified using silica gel column chromatography using EtOAc and heptane (10-30%) solvents yielding 7-cyclopropyl-4-methoxythieno[3,2-d]pyrimidine **26** as a light yellow solid (3 g, 90% yield).

Compound **27** was synthesized using General Synthetic Method **A.**

**Synthesis of 28:** 6-chloro-7-cyclopropyl-4-methoxythieno[3,2-d]pyrimidine (2 g, 8.25 mmol) was taken in 12 N HCl (6 mL, 72.5 mmol) and heated the reaction mixture at 70°C for 40 minutes. After cooling it to the room temperature, water (15 mL) was added to this pale brown slurry and mixed properly. The precipitate was filtered off and washed it with water and heptanes. Upon drying it for overnight at 40°C afforded a light yellow solid (1.6 g, 90% yield)

Compound **29** was synthesized using General Synthetic Method **B.** Compound **30** was synthesized using General Synthetic Method **B. MS** *m*/*z* C₁₁H₉ClN₂O₂S₂ [M+1]⁺ = 301 and [M+1]⁺³ = 303. **¹H NMR** (400 MHz, DMSO-d₆) δ ppm 8.92 (s, 1 H), 4.22 (s, 2 H), 2.10 - 2.20 (m, 1 H), 1.45 - 1.52 (m, 2 H), 0.96 - 1.06 (m, 2 H). **¹³C NMR** (101 MHz, DMSO-*d*₆) δ ppm 169.6 (s, 2C), 161.4 (s, 1C), 156.2 (s, 1C), 154.1(s, 2C), 134.4 (s, 1C), 125.9 (s, 1C), 31.9 (s, 1C), 10.2 (s, 1C), 6.3 (s, 2C).

### 6.14. General Synthetic Method K: Preparation of 2-(7-cyclopropyl-6-methylthieno[2,3-d]pyrimidin-4-ylthio)acetic acid (35)

This general approach to the synthesis of compounds encompassed by the invention is illustrated by the specific example described below:

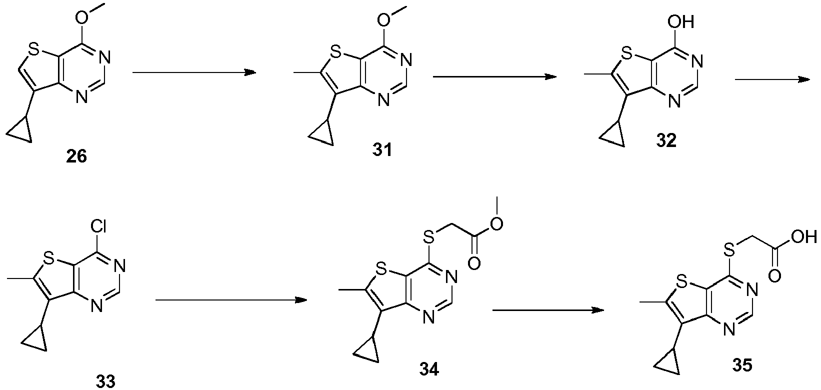

Compound **26** was synthesized using General Synthetic Method J.

**Synthesis of 31:** To the N,N-diisopropyl ethyl amine (14 mL, 80 mmol) in THF (60 mL) nBuLi (37.5 mL, 1.6 M, 60 mmol) was added drop wise at -60°C. After 15 minutes stirring at the same temperature compound **31** (4.14 g, 20 mmol) was added in one portion and allowed to warm up to - 45°C in 45 minutes. Then it was cooled back to -60°C and Mel (5 mL, 80 mmol) was quenched by slow drop wise addition, and stirred for 2 hrs by warming up the reaction mixture to room temperature. After 2 hrs it was quenched with brine and water (100 mL) mixture and separated the THF layer. The aqueous layer was extracted with dichloromethane and the combined organic solvents were washed again with brine and passed through MgSO₄. The crude product was purified using silica gel chromatography using ethyl acetate / hexanes mixture (10% - 30 %) afforded (2.64 g, 60% yield) the compound **32.**

Compounds **32-35** were synthesized using General Synthetic Method **B. MS** *m*/*z* C₁₂H₁₂N₂O₂S₂ [M+1]⁺ = 281. **¹H NMR** (400 MHz, DMSO-d₆) δ ppm 12.86 (br. s., 1 H), 8.85 (s, 1 H), 4.20 (s, 2 H), 2.65 (s, 3 H), 1.99 (tt, *J*=8.63, 5.43 Hz, 1 H), 1.23 - 1.30 (m, 2 H), 0.88 - 0.97 (m, 2 H).

### 6.15. General Synthetic Method L: Preparation of 4-(carboxymethylthio)-7-methylthieno[3,2-d]pyrimidine-6-carboxylic acid (40)

This general approach to the synthesis of compounds encompassed by the invention is illustrated by the specific example described below:

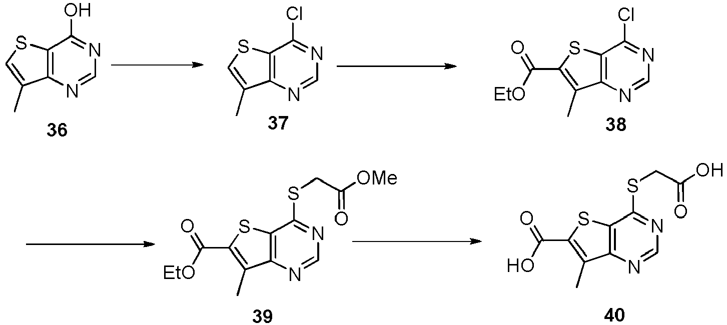

Compound **36** is commercially available. Compound **37** was synthesized using General Synthetic Method **B.**

**Synthesis of 38:** To a stirred solution of thiophene **37** (368 mg, 2 mmol, 1.0 eq.) in anhydrous THF (5 mL) at -30 °C was added a LDA solution (2.2 mmol, 1.1 eq) in THF (freshly prepared by mixing 0.34 mL of diisopropylamine and 0.96 mL 2.5M nBuLi at -30 °C in 5 mL of THF). The reaction turned from clear to yellow. After 20 minutes, ethylchloroformate (0.28 mL, 3 mmol, 1.5 eq) was added drop wise. The reaction was quenched by water after it was warmed up to rt., in the cold bath. The mixture was diluted with 10 mL ethyl acetate, extracted with ethyl acetate (10 mL x 2 times). The combined organic layer was washed with brine, dried over Na₂SO₄, concentrated. The crude mixture was then absorbed on silica gel and purified by flash chromatography (eluted by 10% DCM/EA) to offer ester **38** as a white solid (127mg, 25% yield).

Compounds **39** and **40** were synthesized using General Synthetic Method **B**.

### 6.16. General Synthetic Method M: Preparation of 2-(6-cyano-7-methylthieno[3,2-d]pyrimidin-4-ylthio)acetic acid (44) and 2-(6-carbamoyl-7-methylthieno[3,2-d]pyrimidin-4-ylthio)acetic acid (46)

This general approach to the synthesis of compounds encompassed by the invention is illustrated by the specific examples described below:

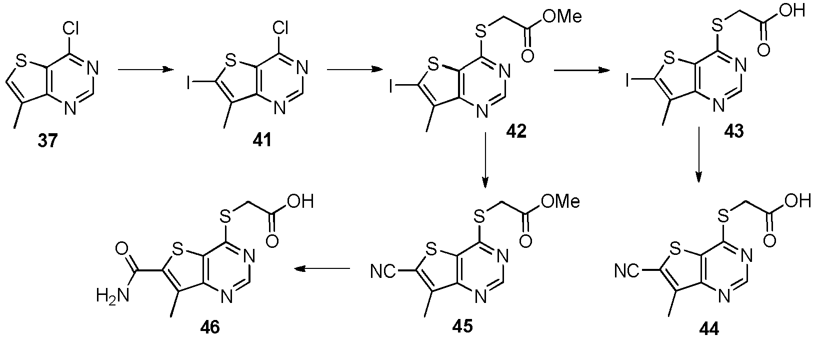

**Synthesis of 41:** To a solution of **37** (368 mg, 2 mmol, 1.0 eq.), prepared using General Synthetic Method **B,** in anhydrous THF (5 mL) at -30°C was added a LDA solution (2.2 mmol, 1.1 eq) in THF (freshly prepared by mixing 0.34 mL of diisopropylamine and 0.96 mL 2.5M nBuLi at -30 °C in 5 mL of THF). The reaction turned from clear to yellow. After 20 mins, iodine (609 mg, 2.4 mmol, 1.2 eq) was added drop wise. The reaction was quenched by water after it was warmed up to room temperature, in the cold bath. The reaction was diluted with 10 mL ethyl acetate, extracted with ethyl aceate (10 mL x 2 ). The combined organic layer was washed with brine, dried over Na₂SO₄ and concentrated. The crude mixture was then absorbed on silica gel and purified by flash chromatography (eluted by 10% Hexane/Ethyl acetate) to afford compound **41** as a white solid (407 mg, 65% yield).

Compounds **42** and **43** were synthesized using General Synthetic Method **B.**

**Synthesis of 44:** Compound **43** (70 mg, 0.19 mmol, 1 eq.) in DMF (1 mL) was added Pd(PPh₃)₄ (44 mg, 0.038 mmol, 0.2 eq.) and Zn(CN)₂ (30mg, 0.25 mmol, 1.1 eq). The vial was degassed with N₂ for 5 minutes before it was capped. The reaction was then heated under microwave irradiation at 100°C for 1.5 hours. All the solvent was removed and the residue was purified by preparative thin layer chromatography gave a white solid (28 mg, 42% yield). **MS** *m*/*z* C₁₀H₇N₃O₂S₂ [M+1]⁺ = 266.0; **¹H NMR** (400 MHz, DMSO-d₆) δ ppm 9.09 (s, 1 H), 4.12 (br. s., 2 H)2.60(s,3H).

**Synthesis of 46:** Compound **45** (40 mg, 0.142 mmol, 1 eq.), prepared using General Synthetic Method **M,** was taken up in 1 mL of MeOH. To this solution was added 1N NaOH (2.1 mL). The resulting mixture was then stirred at room temperature before it was neutralized by adding 1N HCl. The reaction was purified by preparative HPLC to offer compound **46** as a white solid (12 mg, 29% yield). **MS** *m*/*z* C₁₀H₉N₃O₃S₂ [M+1]⁺ = 284.0, observed 224. **¹H NMR** (400 MHz, CHLOROFORM-d) δ ppm 8.83 (s, 1 H) 6.06 - 6.36 (m, 2 H) 4.20 (s, 2 H) 2.77 (s, 3 H).

### 6.17. General Synthetic Method N: Preparation of 2-(3-choloro-2-methylthieno[3,2-c]pyridin-4-ylthio)acetic acid (50)

This general approach to the synthesis of compounds encompassed by the invention is illustrated by the specific example described below: 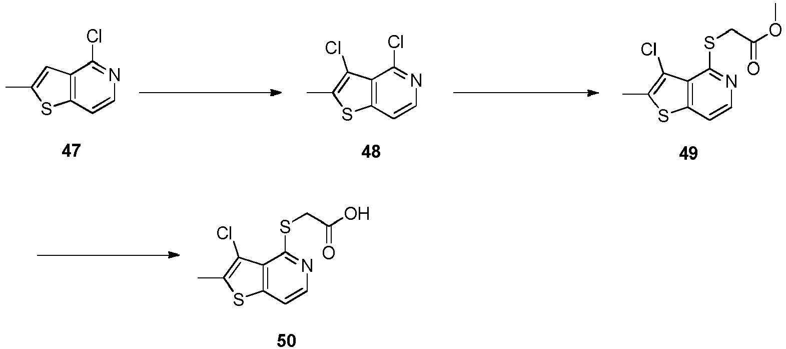 Compound **47** is commercially available. Compound **48** was synthesized using General Method A. Compounds **49** and **50** were synthesized using General Method B.

### 6.18. General Synthetic Method O: Preparation of 2-(5,6-dimethylfuro[2,3-d]pyridin-4-ylthio)acetic acid (55)

This general approach to the synthesis of compounds encompassed by the invention is illustrated by the specific example described below:

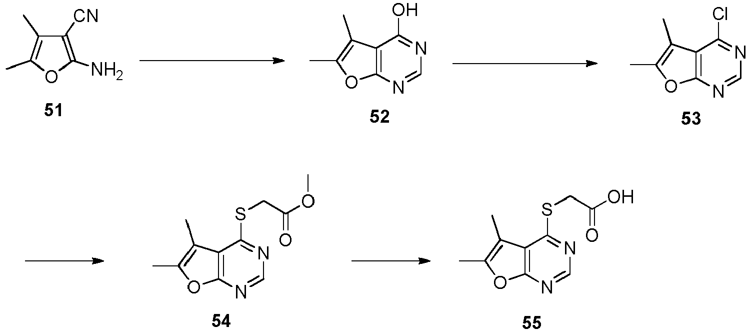

**Synthesis of 52:** In a 50 mL round bottom flask 2-amino-4,5-dimethylfuran-3-carbonitrile **51** (1g, 7.4 mmol), was taken up in formic acid (15 mL) as a solvent. This mixture was heated to reflux temperature for 6 hours. The reaction was cooled to room temperature and concentrated under vacuum pump. Then it was taken up in dichloro methane washed with H₂O. Then the crude product was passed through Na₂SO₄ and concentrated to obtain the crude dimethylfuro pyrimidinol **52** as a crude product, which is directly used in the next step.

**Synthesis of 53:** 5,6-dimethylfuro[2,3-d]pyrimidin-4-ol **52** (700 mg, 4.3 mmol, 1 eq.) was suspended in POCl₃ (10 mL, 100 mmol, 23 eq.) and heated to 90°C for 3 hours. After cooling the reaction was concentrated and the residue cooled to 0°C. Ice cold water was then added slowly to ensure complete consumption of POCl₃. The black solution was thrice extracted with ethyl acetate. The combined organic extracts were twice washed with saturated aqueous NaHCO₃, once with brine, dried over MgSO₄, filtered and concentrated, yielding light brown solid 4-chloro-5,6-dimethylfuro[2,3-d]pyrimidine **53** (350 mg, 55% yield), which was immediately used after complete drying.

**Synthesis of 54:** 4-chloro-5,6-dimethylfuro[2,3-d]pyrimidine **53** (350 mg, 1.91 mmol, 1 eq.) was suspended in methanol (10 mL) and cooled to 0°C. Methyl 2-mercaptoacetate (0.13 mL, 2.0 mmol, 1.05 eq.) was added slowly, followed by the slow addition of TEA (0.39 mL, 3.84 mmol, 2.1 eq.). After stirring for 90 minutes, the reaction was concentrated. The residue was taken up in ethyl acetate and filtered through a silica gel plug to remove salts and some color. The filtrate was concentrated and purified using silica gel chromatography with 0-15% ethyl acetate in hexanes, yielding methyl 2-(5,6-dimethylfuro[2,3-d]pyrimidin-4-ylthio)acetate **54** (420 mg, 90% yield) as a solid.

**Synthesis of 55:** Methyl 2-(5,6-dimethylfuro[2,3-d]pyrimidin-4-ylthio)acetate **54** (400 mg, 1.58 mmol, 1 eq.) was taken up in THF (10 mL) and 1 N aqueous NaOH (3.2 mL, 3.2 mmol, 2 eq.) was added, with efficient stirring. After 40 minutes, the reaction was concentrated and the residue taken up in water, cooled to 0°C and acidified to low pH with 1 N HCl. The precipitate was collected via vacuum filtration, washed with water. Drying it under vacuum for overnight, yielded pure 2-(5,6-dimethylfuro[2,3-d]pyrimidin-4-ylthio)acetatic acid **55** (353 mg, 94% yield) as a solid.

### 6.19. General Synthetic Method P: Preparation of 2-(7-cyclopropyl-6-(trifluoromethyl)thieno[3,2-d]pyrimidin-4-yl)thio)acetic acid (60):

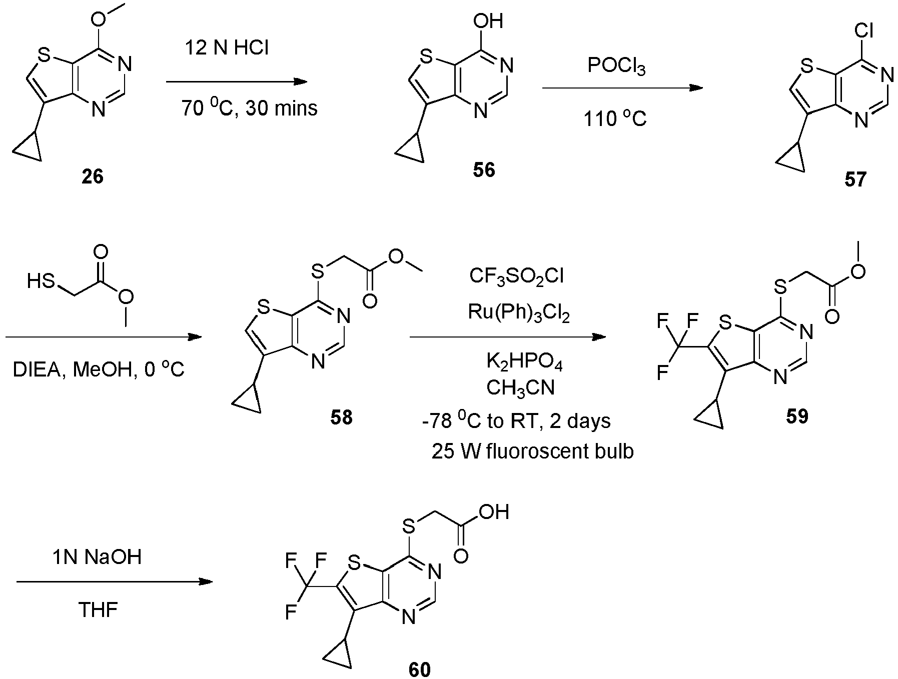

Compounds **26, 56, 57, 58** and **60** were synthesized using General Synthetic Method J.

**Synthesis of 59:** In a 100 mL round bottom flask equipped with a Ru(phen)₃C!₂ (14 mg, 0.01 equiv), and K₂HPO₄ (930 mg, 3 equiv) and the reaction mixture was degassed through alternating vacuum evacuation and nitrogen backfill (x3) before MeCN (20 mL, 0.125 M) and ethyl 2-(7-cyclopropylthieno[3,2-d]pyrimidin-4-yl)thio)acetate **58** (500 mg, 1 equiv) were added by syringe. The resulting solution was degassed by alternating vacuum evacuation and nitrogen backfill (x3) at -78 °C, allowing solution to warm to room temperature under nitrogen between each iteration. The triflyl chloride (0.4 mL, 2 equiv) was added by syringe and the vial was sealed with parafilm and placed approximately 2 cm from a 25 W compact fluorescent light bulb. After 48 hours, the reaction was quenched with water (2 mL) and extracted with CH₂Cl₂ (x2), and the combined organic layers were dried over MgSO₄ and concentrated *in vacuo.* The crude material was then purified by prep HPLC using neutral conditions to afford methyl 2-(7-cyclopropyl-6-(trifluoromethyl)thieno[3,2-d]pyrimidin-4-yl)thio)acetate **59** (62 mgs) in 10% yield.

### 6.20. Representative Compounds

Numerous compounds were made and tested for their activity in one or more of the assays described herein. Some of those compounds are listed below in Table 1, in which the column "Prep" indicates the general synthetic method used to make the named compound. The column "HPLC Method & Time (min)" refers to the following HPLC conditions:
**A:** Sunfire C18 5u 4.6 x 50 mm, 10% to 90% B, gradient time = 2 min, flow rate = 3.5 mL/min, wavelength = 220 and 254 nm, solvent A = 10 mM aqueous ammonium acetate, solvent B = acetonitrile.
**B:** Sunfire C18 5u 4.6 x 50 mm, 10% to 90% B, gradient time = 2 min, flow rate = 3.5 mL/min, wavelength = 254 and 280 nm, solvent A = purified water, solvent B = 95% methanol/5% water with 0.1% trifluoroacetic acid (v/v).
The column "IC₅₀" provides the compounds' IC₅₀ as measured using the binding assay described herein, wherein: **** means a value of less than or equal to 0.025 µM; *** means a value of less than or equal to 0.05 µM; ** means a value of less than or equal to 0.1 µM; * means a value of less than or equal to 0.25 µM; and -- means that the IC₅₀ was not determined. The column "EC₅₀" provides the compounds' EC₅₀ as measured using the reporter assay described herein, wherein: **** means a value of less than or equal to 1 µM; *** means a value of less than or equal to 5 µM; ** means a value of less than or equal to 10 µM; * means a value of less than or equal to 15 µM; and -- means that the EC₅₀ was not determined.

**Table 1**

| Compound | **Prep** | **HPLC Method & Time (min)** | **Purity (%)** | **IC₅₀** | **EC₅₀** |
|---|---|---|---|---|---|
| 2-((5,6-dimethythieno[2,3-d]pyrimidin-4-yl)thio)-N-(furan-2-ylmethyl)acetamide | G | A (1.742) | 99 | **** | -- |
| 2-((5,6-dimethylthieno[2,3-d]pyrimidin-4-yl)thio)acetic acid | B | A (1.047) | 99 | **** | **** |
| 2-((5,6-dimethythieno[2,3-d]pyrimidin-4-yl)thio)-N-(5-methylisoxazol-3-yl)acetamide | G | A (1.763) | 100 | **** | **** |
| 2-((6,7-dihydro-5H-cyclopenta[4,5]thieno[2,3-d]pyrimidin-4-yl)thio)acetic acid | B | A (1.188) | 100 | **** | *** |
| 2-((5,6-dimethythieno[2,3-d]pyrimidin-4-yl)thio)-N-(2-fluorophenyl)acetamide | G | -- | 90 | **** | -- |
| methyl 2-((5,6-dimethylthieno[2,3-d]pyrimidin-4-yl)thio)acetate | H | A (1.927) | 100 | **** | **** |
| 2-((5,6-dimethythieno[2,3-d]pyrimidin-4-yl)thio)-N-methylacetamide | G | A (1.493) | 98 | **** | **** |
| 1-(2-((5,6-dimethylthieno[2,3-d]pyrimidin-4-yl)thio)acetyl)piperidine-4-carboxamide | G | A (1.397) | 99 | * | -- |
| propyl 2-((5,6-dimethylthieno[2,3-d]pyrimidin-4-yl)thio)acetate | H | A (2.235) | 100 | **** | **** |
| buty 2-((5,6-dimethylthieno[2,3-d]pyrimidin-4-yl)thio)acetate | H | A (2.370) | 100 | **** | **** |
| isopropyl 2-((5,6-dimethylthieno[2,3-d]pyrimidin-4-yl)thio)acetate | H | A (2.217) | 100 | **** | **** |
| 2-((6-methylthieno[2,3-d]pyrimidin-4-yl)thio)acetic acid | B | A (0.915) | 100 | * | ** |
| 2-((7-methylthieno[3,2-d]pyrimidin-4-yl)thio)acetic acid | B | A (0.915) | 100 | * | * |
| 2-((5-methylthieno[2,3-d]pyrimidin-4-yl)thio)acetic acid | B | A (0.925) | 100 | ** | -- |
| 2-((5,6-dimethythieno[2,3-d]pyrimidin-4-yl)thio)-N-(ethylsulfonyl)acetamide | G | A (1.218) | 100 | **** | -- |
| 2-((5,6-dimethythieno[2,3-d]pyrimidin-4-yl)thio)acetamide | G | A (1.440) | 100 | **** | **** |
| 2-((5,6-dimethylthieno[2,3-d]pyrimidin-4-yl)thio)-N-((tetrahydro-2H-pyran-2-yl)oxy)acetamide | G | A (1.760) | 100 | **** | **** |
| N-(cyclopropylsulfonyl)-2-((5,6-dimethylthieno[2,3-d]pyrimidin-4-yl)thio)acetamide | G | A (1.235) | 100 | **** | -- |
| 2-((5,6-dimethythieno[2,3-d]pyrimidin-4-yl)thio)-N-hydroxyacetamide | G | A (1.970) | 100 | **** | **** |
| 2-((5,6-dimethylthieno[2,3-d]pyrimidin-4-yl)thio)-N-((5-methylpyridin-2-yl)sulfonyl)acetamide | G | A (1.273) | 97 | ** | -- |
| 2-((5,6-dimethythieno[2,3-d]pyrimidin-4-yl)thio)-1-(isoindolin-2-yl)ethanone | G | A (1.917) | 99 | **** | ** |
| 2-((5,6-dimethythieno[2,3-d]pyrimidin-4-yl)thio)-N-methoxy-N-methylacetamide | G | A (1.700) | 97 | **** | **** |
| 2-((5,6-dimethythieno[2,3-d]pyrimidin-4-yl)thio)-N-(thiophen-2-ylmethyl)acetamide | G | A (1.820) | 99 | ** | -- |
| 1-(5,6-dimethoxyisoindolin-2-y)-2-((5,6-dimethylthieno[2,3-d]pyrimidin-4-yl)thio)ethanone | G | A (1.745) | 100 | **** | -- |
| N-cyano-2-((5,6-dimethylthieno[2,3-d]pyrimidin-4-yl)thio)acetamide | G | A (1.162) | 98 | **** | **** |
| N-(4-chloro-1H-indazol-3-yl)-2-((5,6-dimethylthieno[2,3-d]pyrimidin-4-yl)thio)acetamide | G | A (1.733) | 100 | **** | -- |
| N-(2-(dimethyamino)ethyl)-2-((5,6-dimethylthieno[2,3-d]pyrimidin-4-yl)thio)acetamide | G | B (1.490) | 99 | -- | -- |
| N-(3-(dimethyamino)propy)-2-((5,6-dimethylthieno[2,3-d]pyrimidin-4-yl)thio)acetamide | G | B (1.522) | 99 | -- | -- |
| 2-((6-ethyl-5-methylthieno[2,3-d]pyrimidin-4-yl)thio)acetic acid | B,E | A (1.212) | 100 | **** | -- |
| 2-((6-bromo-5-methylthieno[2,3-d]pyrimidin-4-yl)thio)acetic acid | B,C | A (1.145) | 100 | **** | **** |
| 2-((5-bromo-6-methylthieno[2,3-d]pyrimidin-4-yl)thio)acetic acid | B | A (1.213) | 99 | **** | **** |
| 2-((6-isopropyl-5-methylthieno[2,3-d]pyrimidin-4-yl)thio)acetic acid | B,D | A (1.305) | 100 | *** | -- |
| methyl 2-((5-cyclopropyl-6-methylthieno[2,3-d]pyrimidin-4-yl)thio)acetate | B,D | A (2.137) | 100 | **** | -- |
| methyl 2-((6-cyclopropyl-5-methylthieno[2,3-d]pyrimidin-4-yl)thio)acetate | B,D | A (2.190) | 100 | **** | -- |
| 2-((5-cyclopropyl-6-methylthieno[2,3-d]pyrimidin-4-yl)thio)acetic acid | B,D | A (1.238) | 100 | **** | *** |
| 2-((6-cyclopropyl-5-methylthieno[2,3-d]pyrimidin-4-yl)thio)acetic acid | B,D | A (1.257) | 99 | -- | *** |
| 2-((5-bromo-6-ethylthieno[2,3-d]pyrimidin-4-yl)thio)acetic acid | B | A (1.408) | 100 | **** | **** |
| 2-((6-bromo-7-methylthieno[3,2-d]pyrimidin-4-yl)thio)acetic acid | B | A (1.267) | 99 | **** | -- |
| 2-((5-bromo-6-isopropylthieno[2,3-d]pyrimidin-4-yl)thio)acetic acid | B | A (1.345) | 100 | **** | **** |
| 2-((5-chloro-6-methylthieno[2,3-d]pyrimidin-4-yl)thio)acetic acid | B | A (1.220) | 92 | **** | **** |
| 2-((6-chloro-5-methylthieno[2,3-d]pyrimidin-4-yl)thio)acetic acid | B | A (1.265) | 100 | **** | -- |
| 2-((6,7-dimethylthieno[3,2-d]pyrimidin-4-yl)thio)acetic acid | B,D | A (1.127) | 99 | **** | -- |
| 2-((6-cyclopropyl-7-methylthieno[3,2-d]pyrimidin-4-yl)thio)acetic acid | B,D | A (1.357) | 98 | -- | -- |
| 2-((6-chloro-7-methylthieno[3,2-d]pyrimidin-4-yl)thio)acetic acid | B | A (1.138) | 100 | **** | **** |
| 2-((7-chloro-6-methylthieno[3,2-d]pyrimidin-4-yl)thio)acetic acid | B | A (1.052) | 100 | -- | -- |
| 2-((5-(dimethylamino)-6-methylthieno[2,3-d]pyrimidin-4-yl)thio)acetic acid | B,F | A (1.088) | 100 | -- | -- |
| methyl 2-((6-chloro-7-methylthieno[3,2-d]pyrimidin-4-yl)thio)acetate | B | A (1.983) | 100 | **** | **** |
| 2-((7,9-dimethylpyrido[3',2':4,5]thieno[3,2-d]pyrimidin-4-yl)thio)acetic acid | B | A (1.320) | 100 | -- | -- |
| 2-(pyrido[3',2':4,5]thieno[3,2-d]pyrimidin-4-ylthio)acetic acid | B | A (1.045) | 100 | -- | -- |
| methyl 2-(pyrido[3',2':4,5]thieno[3,2-d]pyrimidin-4-ylthio)acetate | B | A (1.770) | 100 | -- | -- |
| 2-((5-chloro-6-ethythieno[2,3-d]pyrimidin-4-yl)thio)acetic acid | B | A (1.220) | 100 | **** | **** |
| 2-((5-chloro-6-isopropylthieno[2,3-d]pyrimidin-4-yl)thio)acetic acid | I | A (1.325) | 100 | **** | **** |
| 2-((6-chloro-7-methylthieno[3,2-d]pyrimidin-4-yl)thio)-N-(2-cyanoethyl)acetamide | G | A (1.893) | 100 | **** | **** |
| 2-((6-chloro-7-methylthieno[3,2-d]pyrimidin-4-yl)thio)-N-(2-methoxyethyl)acetamide | G | A (1.937) | 100 | **** | **** |
| 2-((6-chloro-7-methylthieno[3,2-d]pyrimidin-4-yl)thio)-N-(2-(piperidin-1-yl)ethyl)acetamide | G | A (1.663) | 100 | -- | **** |
| 2-((6-chloro-7-methylthieno[3,2-d]pyrimidin-4-yl)thio)-N-(2-methyl-2-morpholinopropyl)acetamide | G | A (2.123) | 100 | -- | -- |
| 2-((6-chloro-7-methylthieno[3,2-d]pyrimidin-4-yl)thio)-N-(2-methyl-2-(pyrrolidin-1-yl)propyl)acetamide | G | A (1.638) | 99 | -- | -- |
| 2-((5-chloro-6-methylthieno[2,3-d]pyrimidin-4-yl)thio)-N-(2-methoxyethyl)acetamide | G | A (1.687) | 96 | -- | -- |
| 2-((5-chloro-6-methylthieno[2,3-d]pyrimidin-4-yl)thio)-N-(2-(piperidin-1-yl)ethyl)acetamide | G | A (1.658) | 100 | -- | -- |
| 2-((5-chloro-6-methylthieno[2,3-d]pyrimidin-4-yl)thio)-N-(2-cyanoethyl)acetamide | G | A (1.805) | 100 | **** | **** |
| 2-((6-chloro-7-methylthieno[3,2-d]pyrimidin-4-yl)thio)-N-ethylacetamide | G | A (2.008) | 100 | **** | **** |
| 2-((6-chloro-7-methylthieno[3,2-d]pyrimidin-4-yl)thio)-N,N-dimethylacetamide | G | A (2.020) | 100 | -- | -- |
| 2-((6-chloro-7-methylthieno[3,2-d]pyrimidin-4-yl)thio)-N-(2-morpholinoethyl)acetamide | G | A (1.818) | 98 | -- | -- |
| 2-((6-chloro-7-methylthieno[3,2-d]pyrimidin-4-yl)thio)-N-propylacetamide | G | A (2.150) | 99 | -- | -- |
| 2-((5-chloro-6-methylthieno[2,3-d]pyrimidin-4-yl)thio)-N-ethylacetamide | G | A (1.918) | 100 | **** | **** |
| 2-((5-chloro-6-methylthieno[2,3-d]pyrimidin-4-yl)thio)-N-(2-methyl-2-(piperidin-1-yl)propyl)acetamide | G | A (1.580) | 99 | -- | -- |
| 2-((5-chloro-6-methylthieno[2,3-d]pyrimidin-4-yl)thio)-N-propylacetamide | G | A (2.105) | 100 | **** | -- |
| 2-((5-chloro-6-methylthieno[2,3-d]pyrimidin-4-yl)thio)-N-(2-morpholinoethyl)acetamide | G | A (1.778) | 99 | **** | **** |
| 2-((5-chloro-6-methylthieno[2,3-d]pyrimidin-4-yl)thio)-N,N-dimethylacetamide | G | A (2.523) | 100 | **** | **** |
| 2-((5-chloro-6-methylthieno[2,3-d]pyrimidin-4-yl)thio)-N-(4-methoxybenzyl)acetamide | G | A (2.172) | 100 | -- | -- |
| 2-((5-chloro-6-methylthieno[2,3-d]pyrimidin-4-yl)thio)-N-(pyridin-4-ylmethyl)acetamide | G | A (1.768) | 99 | **** | -- |
| 2-((5-chloro-6-methylthieno[2,3-d]pyrimidin-4-yl)thio)-N-(thiophen-2-ylmethyl)acetamide | G | A (2.155) | 100 | **** | -- |
| 2-((5-chloro-6-methylthieno[2,3-d]pyrimidin-4-yl)thio)-N-(2-(4-methylthiazol-2-yl)ethyl)acetamide | G | A (1.968) | 99 | **** | -- |
| 2-((6-chloro-7-methylthieno[3,2-d]pyrimidin-4-yl)thio)-1-(4,4-difluoropiperidin-1-yl)ethanone | G | A (2.320) | 100 | -- | -- |
| N-(2-(1H-pyrazol-4-y)ethyl)-2-((5-chloro-6-methylthieno[2,3-d]pyrimidin-4-yl)thio)acetamide | G | A (1.712) | 100 | *** | **** |
| 2-((5-chloro-6-methylthieno[2,3-d]pyrimidin-4-yl)thio)-N-(4-cyanobenzyl)acetamide | G | A (2.107) | 100 | -- | -- |
| 2-((5-chloro-6-methylthieno[2,3-d]pyrimidin-4-yl)thio)-N-(4-fluorobenzyl)acetamide | G | A (2.203) | 100 | -- | -- |
| 2-((5-chloro-6-methylthieno[2,3-d]pyrimidin-4-yl)thio)-N-(furan-2-ylmethyl)acetamide | G | A (2 083) | 100 | **** | **** |
| 2-((6-chloro-7-methylthieno[3,2-d]pyrimidin-4-yl)thio)-N-(4-methoxybenzyl)acetamide | G | A (2.257) | 99 | -- | -- |
| 2-((6-chloro-7-methylthieno[3,2-d]pyrimidin-4-yl)thio)-N-(4-fluorobenzyl)acetamide | G | A (1.970) | 97 | -- | -- |
| 2-((6-chloro-7-methylthieno[3,2-d]pyrimidin-4-yl)thio)-N-(thiophen-2-ylmethyl)acetamide | G | A (2.247) | 100 | -- | **** |
| methyl 2-(2-((6-chloro-7-methylthieno[3,2-d]pyrimidin-4-yl)thio)acetyl)isoindoline-4-carboxylate | G | A (2.420) | 100 | -- | -- |
| N-(2-(1H-imidazol-4-y)ethyl)-2-((5-chloro-6-methylthieno[2,3-d]pyrimidin-4-yl)thio)acetamide | G | A (1.532) | 100 | **** | -- |
| 2-((5-chloro-6-methylthieno[2,3-d]pyrimidin-4-yl)thio)-N-(cyanomethyl)acetamide | G | A (1.930) | 100 | **** | **** |
| methyl 2-((6-(tert-butyl)thieno[3,2-d]pyrimidin-4-yl)thio)acetate | B | A (2.375) | 100 | -- | -- |
| methyl 2-((6-cyano-7-methylthieno[3,2-d]pyrimidin-4-yl)thio)acetate | | B (2.498) | 97 | -- | **** |
| 2-((6-cyano-7-methylthieno[3,2-d]pyrimidin-4-yl)thio)acetic acid | M | A (1.330) | 96 | -- | **** |
| 2-((6-chloro-7-methylthieno[3,2-d]pyrimidin-4-yl)thio)-N-(cyanomethyl)acetamide | G | A (2.003) | 99 | **** | **** |
| isopropyl 2-((5-chloro-6-methylthieno[2,3-d]pyrimidin-4-yl)thio)acetate | H | A (2.587) | 100 | **** | **** |
| 2-((6-chloro-7-methylthieno[3,2-d]pyrimidin-4-yl)thio)-N-(1-methylpiperidin-4-yl)acetamide | G | A (1.605) | 100 | -- | -- |
| 2-((5-chloro-6-methylthieno[2,3-d]pyrimidin-4-yl)thio)-1-(4-isopropylpiperazin-1-yl)ethanone | G | A (1.947) | 100 | -- | -- |
| 2-((5-chloro-6-methylthieno[2,3-d]pyrimidin-4-yl)thio)-N-(1-methylpiperidin-4-yl)acetamide | G | A (1.557) | 99 | -- | -- |
| 2-(2-((5-chloro-6-methylthieno[2,3-d]pyrimidin-4-yl)thio)-N-methylacetamido)acetic acid | G,B | A (1.648) | 100 | -- | **** |
| 2-((5-chloro-6-methylthieno[2,3-d]pyrimidin-4-yl)thio)-N-(methylsulfonyl)acetamide | G | A (1.522) | 100 | -- | **** |
| 2-((6-chloro-7-cyclopropylthieno[3,2-d]pyrimidin-4-yl)thio)acetic acid | J | A (1.927) | 97 | **** | **** |
| 2-((5-chloro-6-methylthieno[2,3-d]pyrimidin-4-yl)thio)-N-(1-hydroxypropan-2-yl)acetamide | G | B (2.543) | 100 | -- | *** |
| 2-((5-chloro-6-methylthieno[2,3-d]pyrimidin-4-yl)thio)-N-(1,3-dihydroxypropan-2-yl)acetamide | G | A (1.623) | 99 | -- | -- |
| (S)-methyl 1-(2-((5-chloro-6-methylthieno[2,3-d]pyrimidin-4-yl)thio)acetyl)pyrrolidine-2-carboxylate | G | A (2.173) | 99 | -- | -- |
| 2-((5-chloro-6-propythieno[2,3-d]pyrimidin-4-yl)thio)acetic acid | B | A (1.452) | 97 | **** | **** |
| methyl 2-((6-chloro-7-cyclopropylthieno[3,2-d]pyrimidin-4-yl)thio)acetate | J | A (2.858) | 100 | **** | -- |
| 2-((7-cyclopropyl-6-methylthieno[3,2-d]pyrimidin-4-yl)thio)acetic acid | K | A (1.305) | 100 | **** | **** |
| 2-((3-chloro-2-methylthieno[3,2-c]pyridin-4-yl)thio)acetic acid | -- | -- | -- | -- | *** |
| 2-(7-cyclopropyl-6-(trifluoromethyl)thieno[3,2-d]pyrimidin-4-yl)thio)acetic acid | J,P | A (1.665) | 96 | **** | **** |
| 2-(7-cyclopropyl-6-(trifluoromethyl)thieno[3,2-d]pyrimidin-4-yl)thio)acetate | J,P | A (2.495) | 95 | **** | **** |

### 6.21. Pharmacology of 2-((5,6-dimethylthieno[2,3-d]pyrimidin-4-yl)thio)acetic acid

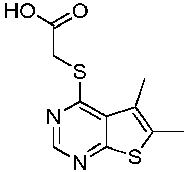

In a first study, the *in vivo* effect of 2-((5,6-dimethylthieno[2,3-d]pyrimidin-4-yl)thio)acetic acid was determined by treating F1 male hybrid (129 x C57) mice for 25 days with the compound, starting at 10.7 weeks of age. The compound was administered in the animals' diet. Four groups of mice were used: control (N = 12); 25 mg/kg compound (N = 9); 83 mg/kg compound (N = 9); and an additional group wherein Li₂CO₃ was administered in the diet (0.1%) (N = 9).

Midshaft femur cortical thickness was measured using a Scanco µCT40. Compared to the control group, an increase in cortical bone thickness was observed at both doses of the compound: 9% (p = 0.04) at 25 mg/kg; and 8% (p = 0.07) at 83 mg/kg. For the Li₂CO₃ group, a decrease in cortical bone thickness of 2% (p = 0.90) was observed.

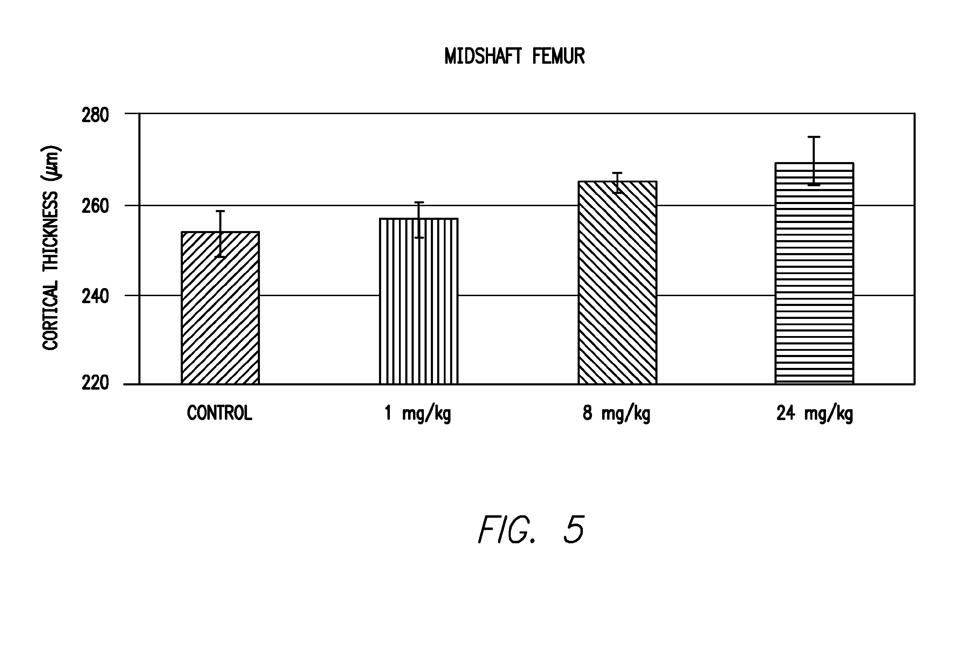

In a second study, the *in vivo* effect of 2-((5,6-dimethylthieno[2,3-d]pyrimidin-4-yl)thio)acetic acid was determined by treating F1 male hybrid (129 x C57) mice for 25 days with the compound, again starting at 10.7 weeks of age. The compound was administered in the animals' diet. Four groups of mice were used: control (N = 12); 1 mg/kg compound (N = 9); 8 mg/kg compound (N = 9); and 24 mg/kg compound (N = 9). As shown in Figure 5, mice treated with 8 mg/kg exhibited a 4% (p = 0.19) increase in midshaft femur cortical thickness, and mice treated with 24 mg/kg exhibited a 6% (p = 0.05) increase.

### 6.22. Pharmacology of 2-((5-chloro-6-methylthieno[2,3-d]pyrimidin-4-yl)thio)acetic acid

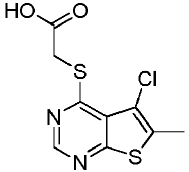

The pharmacology of 2-((5-chloro-6-methylthieno[2,3-d]pyrimidin-4-yl)thio)acetic acid was studied in Fischer 344 ovariectomized rats. The rats underwent ovariectomies or sham surgery at 43 or 46 weeks of age, and treatment with the compound started at 37 or 40 weeks thereafter, at 83 weeks of age.

Four treatment groups were used: sham-surgery given control diet (N = 14); sham-surgery given compound (N = 12); OVX-surgery given control diet (N = 14); and OVX-surgery given compound (N = 12). The compound was dosed by incorporating it into the rats' diet: 0.46 grams of compound per kg of diet. The target dose was 30 mg/kg. Dosing occurred for five weeks.

Bone mass and architecture was determined by microCT, using a Scanco µCT40. As shown in Figure 6, treatment increased midshaft femur cortical bone thickness in both the sham and OVX groups: 3% in intact rats and 5% in ovariectomized rats. The difference between the cortical bone thicknesses of the sham control and OVX control was -13%. Two-factor ANOVA values were: p < 0.001 for OVX surgery; p = 0.05 for treatment; and p = 0.65 for interaction.

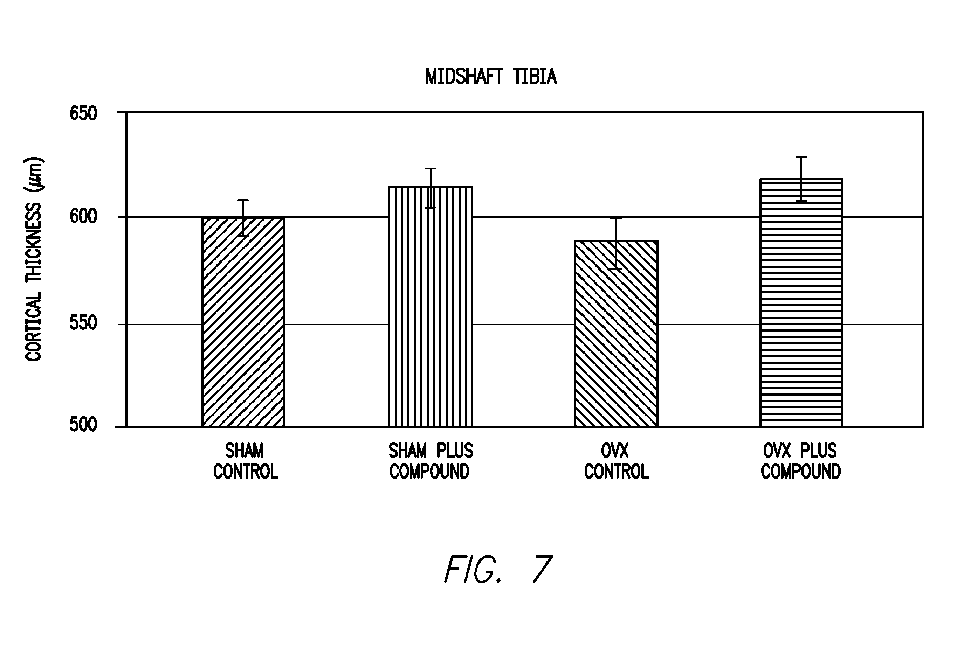

As shown in Figure 7, treatment also increased midshaft tibia cortical thickness in both the sham and OVX groups: 2% in intact rats and 5% in ovariectomized rats. Two-factor ANOVA values were: p = 0.79 for OVX surgery; p = 0.05 for treatment; and p = 0.52 for interaction.

### 6.23. Pharmacology of 2-((5-chloro-6-isopropylthieno[2,3-d]pyrimidin-4-yl)thio)acetic acid

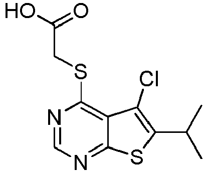

The in vivo effect of 2-((5-chloro-6-isopropylthieno[2,3-d]pyrimidin-4-yl)thio)acetic acid was determined by treating F1 male hybrid (129 x C57) mice for 25 days with the compound, starting at 8.7 weeks of age. The compound was administered twice daily by oral gavage (vehicle = 0.1% Tween 80 in water). Four groups of mice were used: control (N = 13); 5 mg/kg compound (N = 13); 10 mg/kg compound (N = 13); 15 mg/kg compound (N = 13).

Midshaft femur cortical thickness was measured by microCT (Scanco µCT40). Compared to the control group, an increase in cortical bone thickness was observed at all doses: 6% (p = 0.002) at 5 mg/kg; 5% (p = 0.007) at 10 mg/kg; and 6% (p = 0.001) at 15 mg/kg.

### 6.24. Pharmacology of 2-((6-chloro-7-cyclopropylthieno[3,2-d]pyrimidin-4-yl)thio)acetic acid

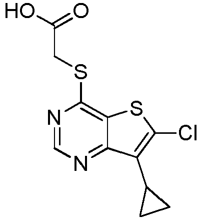

The *in vivo* effect of 2-((6-chloro-7-cyclopropylthieno[3,2-d]pyrimidin-4-yl)thio)acetic acid was determined by treating F1 male hybrid (129 x C57) mice for 25 days with the compound, starting at 8.7 weeks of age. The compound was administered by daily oral gavage (vehicle = 0.1% Tween 80 in water). Four groups of mice were used: control (N = 13); 3 mg/kg compound (N = 13); 10 mg/kg compound (N = 13); 30 mg/kg compound (N = 13).

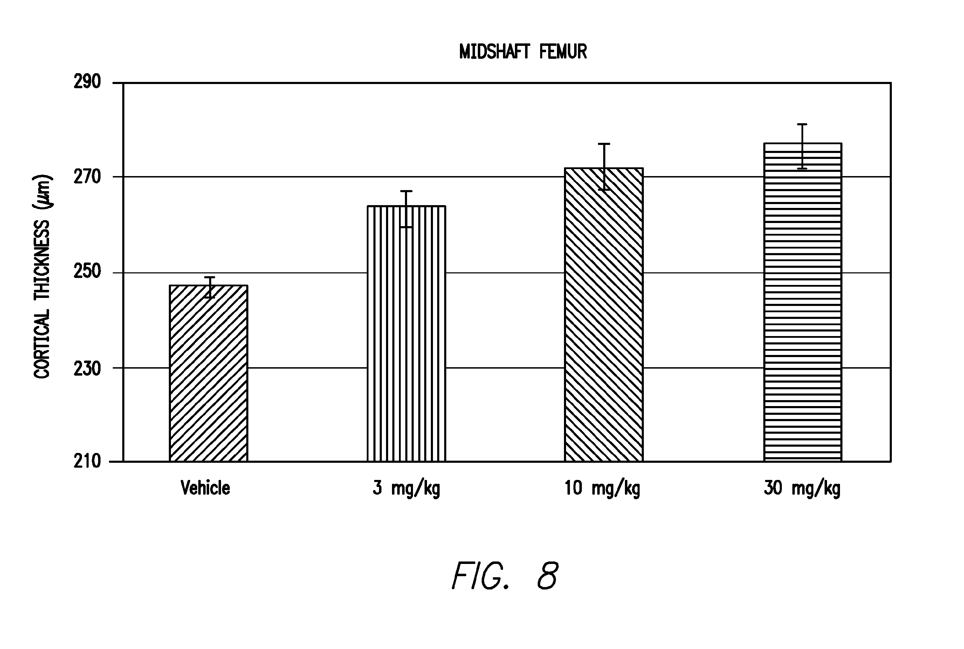

Midshaft femur cortical thickness was measured by microCT (Scanco µCT40). As shown in Figure 8, an increase in cortical bone thickness was observed at all doses compared to control: 7% (p = 0.003) at 3 mg/kg; 10% (p < 0.001) at 10 mg/kg; and 13% (p < 0.001) at 30 mg/kg.

### 6.25. Pharmacology of 2-((6-chloro-7-methylthieno[3,2-d]pyrimidin-4-yl)thio)acetic acid

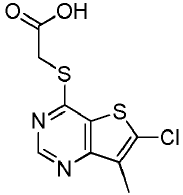

In a first experiment, the *in vivo* effect of 2-((6-chloro-7-methylthieno[3,2-d]pyrimidin-4-yl)thio)acetic acid was determined by treating F1 male hybrid (129 x C57) mice for 25 days with the compound, starting at 54 weeks of age. The compound was administered by daily oral gavage (vehicle = 0.1% Tween 80 in water). Three groups of mice were used: control (N = 10); 10 mg/kg compound (N = 10); and 100 mg/kg compound (N = 10). An increase in midshaft femur cortical bone thickness, as measured by microCT (Scanco µCT40), was observed at both doses compared to control: 6% (p = 0.06) at 10 mg/kg; and 7% (p=0.03) at 100 mg/kg. In this experiment, as was generally the case, midshaft femur cortical thickness data were obtained using the right femur of each mouse. In this experiment, the results for the 10 mg/kg dose group includes a left femur measurement for one of the mice, whose right femur measurements were aberrant.

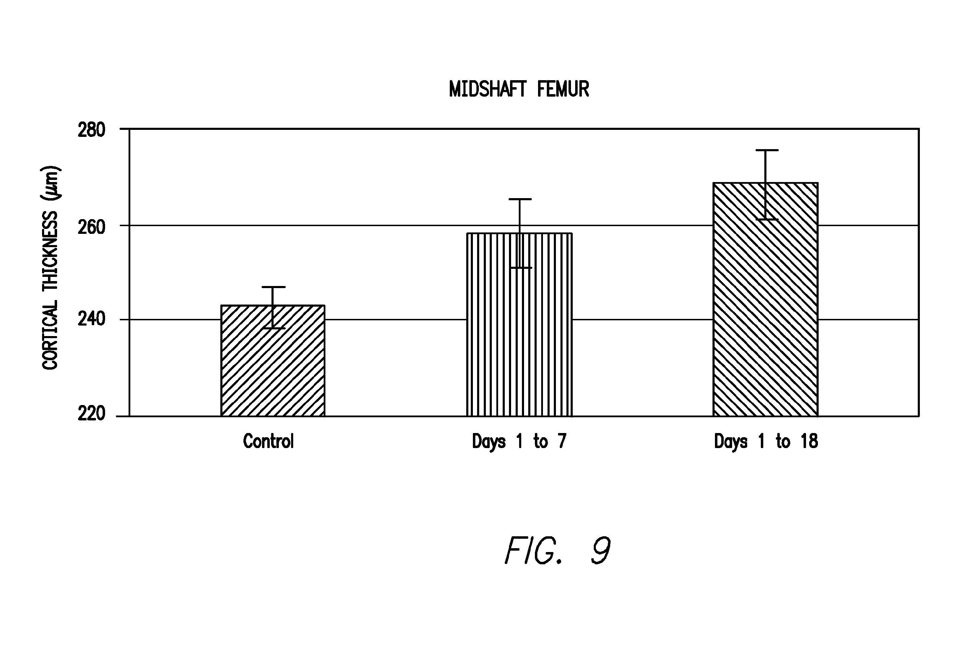

In a second experiment, the *in vivo* effect of 2-((6-chloro-7-methylthieno[3,2-d]pyrimidin-4-yl)thio)acetic acid was determined by treating F1 male hybrid (129 x C57) mice for seven and 18 days with the compound, starting at 8.1 weeks of age. The compound was administered in the animals' diet. Three groups of mice were used: control (N = 9); 34 mg/kg compound for seven days (N = 9); and 34 mg/kg compound for 18 days (N = 9). As shown in Figure 9, an increase in midshaft femur cortical bone thickness was observed in both treatment groups compared to control: 6% (p = 0.13) after seven days of treatment; and 10% (p < 0.01) after 18 days of treatment.

## Claims

1. A compound of the formula: 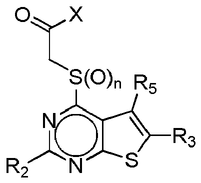 or a pharmaceutically acceptable salt thereof, wherein:
X is OR_{1B} or N(R_{1B})₂;
each R_{1B} is independently hydrogen or optionally substituted alkyl, aryl, heteroalkyl, or heterocycle;
R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle;
each R_{2A} is independently hydrogen or optionally substituted alkyl, aryl, heteroalkyl, or heterocycle;
R₃ is hydrogen, halo, cyano, -CO₂R_{3A}, -C(O)N(R_{3A})₂, -SR_{3A}, -OR_{3A}, -N(R_{3A})₂, or optionally substituted alkyl, aryl, heteroalkyl, or heterocycle;
each R_{3A} is independently hydrogen or optionally substituted alkyl, aryl, heteroalkyl, or heterocycle;
R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle;
each R_{5A} is independently hydrogen or optionally substituted alkyl, aryl, heteroalkyl, or heterocycle; and
n is 1 or 2;
wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

2. A compound of the formula: 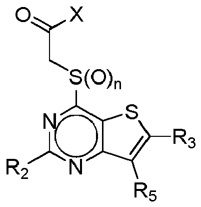 or a pharmaceutically acceptable salt thereof, wherein:
X is OR_{1B} or N(R_{1B})₂;
each R_{1B} is independently hydrogen or optionally substituted alkyl, aryl, heteroalkyl, or heterocycle;
R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle;
each R_{2A} is independently hydrogen or optionally substituted alkyl, aryl, heteroalkyl, or heterocycle;
R₃ is hydrogen, halo, cyano, -CO₂R_{3A}, -C(O)N(R_{3A})₂, -SR_{3A}, -OR_{3A}, -N(R_{3A})₂, or optionally substituted alkyl, aryl, heteroalkyl, or heterocycle;
each R_{3A} is independently hydrogen or optionally substituted alkyl, aryl, heteroalkyl, or heterocycle;
R₄ is hydrogen, halo, or optionally substituted alkyl, aryl, heteroalkyl, or heterocycle;
R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle;
each R_{5A} is independently hydrogen or optionally substituted alkyl, aryl, heteroalkyl, or heterocycle; and
n is 1 or 2;
wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

3. The compound of claim 1 or 2, wherein X is OR_{1B}.

4. The compound of any of claims 1-3, wherein R_{1B} is hydrogen or optionally substituted alkyl or aryl.

5. The compound of claim 4, wherein R_{1B} is hydrogen or alkyl.

6. The compound of any of claims 1-3, wherein R₂ is hydrogen or optionally substituted alkyl.

7. The compound of any of claims 1-3, wherein R₃ is alkyl or halo.

8. The compound of claim 7, wherein R₃ is chloro.

9. The compound of any of claims 1-3, wherein R₅ is alkyl or halo.

10. The compound of claim 9, wherein R₅ is C₁₋₄-alkyl.

11. A compound or a pharmaceutically acceptable salt thereof, which compound is 2-((5,6-dimethylthieno[2,3-d]pyrimidin-4-yl)thio)acetic acid, 2-((5-chloro-6-isopropylthieno[2,3-d]pyrimidin-4-yl)thio)acetic acid, 2-((6-chloro-7-cyclopropylthieno[3,2-d]pyrimidin-4-yl)thio)acetic acid, or 2-((6-chloro-7-methylthieno[3,2-d]pyrimidin-4-yl)thio)acetic acid.

12. A pharmaceutical composition comprising a compound of any of claims 1-11 and a pharmaceutically acceptable excipient or diluent.

13. The compound of any of claims 1-11 for use as a medicament for the treatment or management of a disease or disorder **characterized by** bone loss.

14. A compound of the formula: 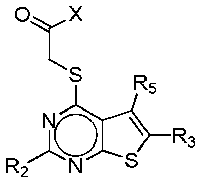 or a pharmaceutically acceptable salt thereof, for use as a medicament for the treatment or management of a disease or disorder **characterized by** bone loss, wherein:
X is OR_{1B} or N(R_{1B})₂;
each R_{1B} is independently hydrogen or optionally substituted alkyl, aryl, heteroalkyl, or heterocycle;
R₂ is hydrogen, halo, -CO₂R_{2A}, -C(O)N(R_{2A})₂, -SR_{2A}, -OR_{2A}, -N(R_{2A})₂, or optionally substituted alkyl, aryl, heteroalkyl, or heterocycle;
each R_{2A} is independently hydrogen or optionally substituted alkyl, aryl, heteroalkyl, or heterocycle;
R₃ is hydrogen, halo, cyano, -CO₂R_{3A}, -C(O)N(R_{3A})₂, -SR_{3A}, -OR_{3A}, -N(R_{3A})₂, or optionally substituted alkyl, aryl, heteroalkyl, or heterocycle;
each R_{3A} is independently hydrogen or optionally substituted alkyl, aryl, heteroalkyl, or heterocycle;
R₅ is hydrogen, halo, -CO₂R_{5A}, -C(O)N(R_{5A})₂, -SR_{5A}, -OR_{5A}, -N(R_{5A})₂, or optionally substituted alkyl, aryl, heteroalkyl, or heterocycle; and
each R_{5A} is independently hydrogen or optionally substituted alkyl, aryl, heteroalkyl, or heterocycle;
wherein "optionally substituted" means optionally substituted with one or more of alkoxy, alkyl, amino (including alkylamino, dialkylamino), aryl, carboxylic acid, cyano, halo, haloalkyl, heterocycle, or hydroxyl.

## Patentansprüche

1. Eine Verbindung der Formel: 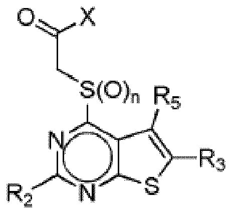 oder ein pharmazeutisch annehmbares Salz davon, wobei:
X OR_{1B} oder N(R_{1B})₂ ist;
jedes R_{1B} unabhängig Wasserstoff oder gegebenfalls substituiertes Alkyl, Aryl, Heteroalkyl oder Heterozyklus ist;
R₂ Wasserstoff, Halogen, -CO₂R_{2A}, -C(O)N(R_{2A})₂, -SR_{2A}, -OR_{2A}, -N(R_{2A})₂, oder gegebenenfalls substituiertes Alkyl, Aryl, Heteroalkyl oder Heterozyklus ist;
jedes R_{2A} unabhängig Wasserstoff oder gegebenenfalls substituiertes Alkyl, Aryl, Heteroalkyl oder Heterozyklus ist;
R₃ Wasserstoff, Halogen,Cyano, -CO₂R_{3A}, -C(O)N(R_{3A})₂, -SR_{3A}, -OR_{3A}, -N(R_{3A})₂, oder gegebenenfalls substituiertes Alkyl, Aryl, Heteroalkyl oder Heterozyklus ist;
jedes R_{3A} unabhängig Wasserstoff oder gegebenenfalls substituiertes Alkyl, Aryl, Heteroalkyl oder Heterozyklus ist;
R₅ Wasserstoff, Halogen, -CO₂R_{5A}, -C(O)N(R_{5A})₂, -SR_{5A}, -OR_{5A}, -N(R_{5A})₂, oder gegebenenfalls substituiertes Alkyl, Aryl, Heteroalkyl oder Heterozyklus ist;
jedes R_{5A} unabhängig Wasserstoff oder gegebenenfalls substituiertes Alkyl, Aryl, Heteroalkyl oder Heterozyklus ist; und
n 1 oder 2 ist;
wobei "gegebenenfalls substituiert" gegebenenfalls substituiert mit ein oder mehr Alkoxy, Alkyl, Amino (einschließlich Alkylamino, Dialkylamino), Aryl, Carbonsäure, Zyano, Halogen, Haloalkyl, Heterozyklus oder Hydroxyl bedeutet.

2. Eine Verbindung der Formel: 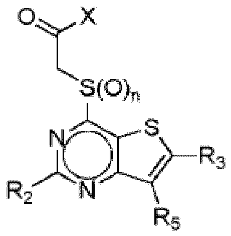 oder ein pharmazeutisch annehmbares Salz davon, wobei:
X OR_{1B} oder N(R_{1B})₂ ist;
jedes R_{1B} unabhängig Wasserstoff oder gegebenenfalls substituiertes Alkyl, Aryl, Heteroalkyl oder Heterozyklus ist;
R₂ Wasserstoff, Halogen, -CO₂R_{2A}, -C(O)N(R_{2A})₂, -SR_{2A}, -OR_{2A}, -N(R_{2A})₂, oder gegebenenfalls substituiertes Alkyl, Aryl, Heteroalkyl oder Heterozyklus ist;
jedes R_{2A} unabhängig Wasserstoff oder gegebenenfalls substituiertes Alkyl, Aryl, Heteroalkyl oder Heterozyklus ist;
R₃ Wasserstoff, Halogen, Zyano, -CO₂R_{3A}, -C(O)N(R_{3A})₂, -SR_{3A}, -OR_{3A}, -N(R_{3A})₂, oder gegebenenfalls substituiertes Alkyl, Aryl, Heteroalkyl oder Heterozyklus ist;
jedes R_{3A} unabhängig Wasserstoff oder gegebenenfalls substituiertes Alky, Aryl, Heteroalkyl oder Heterozyklus ist;
R₄ Wasserstoff, Halogen oder gegebenenfalls substituiertes Alkyl, Aryl, Heteroalkyl oder Heterozyklus ist;
R₅ Wasserstoff, Halogen, -CO₂R_{5A}, -C(O)N(R_{5A})₂, -SR_{5A}, -OR_{5A}, -N(R_{5A})₂, oder gegebenenfalls substituiertes Alkyl, Aryl, Heteroalkyl oder Heterozyklus ist;
jedes R_{5A} unabhängig Wasserstoff oder gegebenenfalls substituiertes Alkyl, Aryl, Heteroalkyl oder Heterozyklus ist; und
n 1 oder 2 ist;
worin "gegebenenfalls substituiert" gegebenenfalls substituiert mit ein oder mehreren Alkoxy, Alkyl, Amino (einschließlich Alkylamino, Dialkylamino), Aryl, Carbonsäure, Zyano, Halogen, Haloalkyl, Heterozyklus oder Hydroxyl bedeutet.

3. Verbindung der Ansprüche 1 oder 2, wobei X OR_{1B} ist.

4. Verbindung nach einem der Ansprüche 1-3, worin R_{1B} Wasserstoff oder gegebenenfalls substituiertes Alkyl oder Aryl ist.

5. Verbindung nach Anspruch 4, wobei R_{1B} Wasserstoff oder Alkyl ist.

6. Verbindung nach einem der Ansprüche 1 bis 3, wobei R₂ Wasserstoff oder gegebenenfalls substituiertes Alkyl ist.

7. Verbindung nach einem der Ansprüche 1 bis 3, worin R₃ Alkyl oder Halogen ist.

8. Verbindung nach Anspruch 7, wobei R₃ Chlor ist.

9. Verbindung nach einem der Ansprüche 1 bis 3, worin R₅ Alkyl oder Halogen ist.

10. Verbindung nach Anspruch 9, worin R₅ C₁₋₄-alkyl ist.

11. Eine Verbindung oder ein pharmazeutisch annehmbares Salz davon, wobei die Verbindung 2-((5,6-dimethylthieno[2,3-d]pyrimidin-4-yl)thio) essigsäure, 2-((5-chloro-6-isopropylthieno[2,3-d]pyrimidin-4yl)thio) essigsäure,2-((6-chloro-7-cyclopropylthieno[3,2-d]pyrimidin-4-yl)thio) essigsäure oder 2-((6-chloro-7-methylthieno [3,2-d]pyrimidin-4-yl)thio) essigsäure ist.

12. Pharmazeutische Zusammensetzung aufweisend eine Verbindung nach einem der Ansprüche 1-11 und einen pharmazeutisch annehmbaren Träger oder Verdünnungsmittel.

13. Verbindung nach einem der Ansprüche 1-11 zur Verwendung als Medikament zur Behandlung oder zum Management einer Krankheit oder Störung, **gekennzeichnet durch** Knochenschwund.

14. Eine Verbindung der Formel: 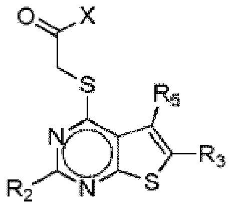 oder ein pharmazeutisch annehmbares Salz davon zur Verwendung als Medikament zur Behandlung oder zum Management einer Krankheit oder Störung, **gekennzeichnet durch** Knochenschwund, wobei:
X OR_{1B} oder N(R_{1B})₂ ist;
jedes R_{1B} unabhängig Wasserstoff oder gegebenfalls substituiertes Alkyl,Aryl, Heteroalkyl oder Heterozyklus ist;
R₂ Wasserstoff, Halogen, -CO₂R_{2A}, -C(O)N(R_{2A})₂, -SR_{2A}, -OR_{2A}, -N(R_{2A})₂, oder gegebenenfalls substituiertes Alkyl, Aryl, Heteroalkyl oder Heterozyklus ist;
jedes R_{2A} unabhängig Wasserstoff oder gegebenenfalls substituiertes Alkyl, Aryl, Heteroalkyl oder Heterozyklus ist;
R₃ Wasserstoff, Halogen, Zyano, -CO₂R_{3A}, -C(O)N(R_{3A})₂, -SR_{3A}, -OR_{3A}, -N(R_{3A})₂, oder gegebenenfalls substituiertes Alkyl, Aryl, Heteroalkyl oder Heterozyklus ist;
jedes R_{3A} unabhängig Wasserstoff oder gegebenenfalls substituiertes Alkyl, Aryl, Heteroalkyl oder Heterozyklus ist;
R₅ Wasserstoff, Halogen, -CO₂R_{5A}, -C(O)N(R_{5A})₂, -SR_{5A}, -OR_{5A}, -N(R_{5A})₂, oder gegebenenfalls substituiertes Alkyl, Aryl, Heteroalkyl oder Heterozyklus ist; und
jedes R_{5A} unabhängig Wasserstoff oder gegebenenfalls substituiertes Alkyl, Aryl, Heteroalkyl oder Heterozyklus ist;
wobei "gegebenenfalls substituiert" gegebenenfalls substituiert mit ein oder mehreren Alkoxy, Alkyl, Amino (einschließlich Alkylamino, Dialkylamino), Aryl, Carbonsäure, Zyano, Halogen, Haloalkyl, Heterozyklus oder Hydroxyl bedeutet.

## Revendications

1. Composé de formule : 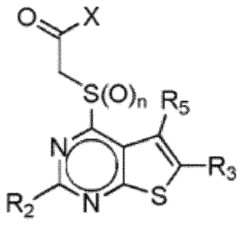 ou un sel pharmaceutiquement acceptable de celui-ci, dans lequel :
X est OR_{1B} ou N(R_{1B})₂,
chaque R_{1B} est indépendamment hydrogène ou alkyle, aryle, hétéroalkyle, ou hétérocycle facultativement substitué ;
R₂ est hydrogène, halogéno, -CO₂R_{2A}, -C(O)N(R_{2A})₂, -SR_{2A}, -OR_{2A}, -N(R_{2A})₂, ou alkyle, aryle, hétéroalkyle, ou hétérocycle facultativement substitué ;
chaque R_{2A} est indépendamment hydrogène ou alkyle, aryle, hétéroalkyle, ou hétérocycle facultativement substitué ;
R₃ est hydrogène, halogéno, cyano, -CO₂R_{3A}, -C(O)N(R_{3A})₂, -SR_{3A}, -OR_{3A}, -N(R_{3A})₂, ou alkyle, aryle, hétéroalkyle, ou hétérocycle facultativement substitué ;
chaque R_{3A} est indépendamment hydrogène ou alkyle, aryle, hétéroalkyle, ou hétérocycle facultativement substitué ;
R₅ est hydrogène, halogéno, -CO₂R_{5A}, -C(O)N(R_{5A})₂, -SR_{5A}, -OR_{5A}, -N(R_{5A})₂, ou alkyle, aryle, hétéroalkyle, ou hétérocycle facultativement substitué ;
chaque R_{5A} est indépendamment hydrogène ou alkyle, aryle, hétéroalkyle, ou hétérocycle facultativement substitué ; et
n est 1 ou 2 ;
où « facultativement substitué » signifie facultativement substitué par l'un ou plusieurs parmi alcoxy, alkyle, amino (comprenant alkylamino, dialkylamino), aryle, acide carboxylique, cyano, halogéno, halogénoalkyle, hétérocycle, ou hydroxyle.

2. Composé de formule : 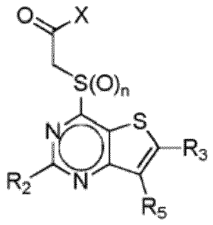 ou un sel pharmaceutiquement acceptable de celui-ci, dans lequel :
X est OR_{1B} ou N(R_{1B})₂,
chaque R_{1B} est indépendamment hydrogène ou alkyle, aryle, hétéroalkyle, ou hétérocycle facultativement substitué ;
R₂ est hydrogène, halogéno, -CO₂R_{2A}, -C(O)N(R_{2A})₂, -SR_{2A}, -OR_{2A}, -N(R_{2A})₂, ou alkyle, aryle, hétéroalkyle, ou hétérocycle facultativement substitué ;
chaque R_{2A} est indépendamment hydrogène ou alkyle, aryle, hétéroalkyle, ou hétérocycle facultativement substitué ;
R₃ est hydrogène, halogéno, cyano, -CO₂R_{3A}, -C(O)N(R_{3A})₂, -SR_{3A}, -OR_{3A}, -N(R_{3A})₂, ou alkyle, aryle, hétéroalkyle, ou hétérocycle facultativement substitué ;
chaque R_{3A} est indépendamment hydrogène ou alkyle, aryle, hétéroalkyle, ou hétérocycle facultativement substitué ;
R₄ est hydrogène, halogéno, ou alkyle, aryle, hétéroalkyle, ou hétérocycle facultativement substitué ;
R₅ est hydrogène, halogéno, -CO₂R_{5A}, -C(O)N(R_{5A})₂, -SR_{5A}, -OR_{5A}, -N(R_{5A})₂, ou alkyle, aryle, hétéroalkyle, ou hétérocycle facultativement substitué ;
chaque R_{5A} est indépendamment hydrogène ou alkyle, aryle, hétéroalkyle, ou hétérocycle facultativement substitué ; et
n est 1 ou 2 ;
où « facultativement substitué » signifie facultativement substitué par l'un ou plusieurs parmi alcoxy, alkyle, amino (comprenant alkylamino, dialkylamino), aryle, acide carboxylique, cyano, halogéno, halogénoalkyle, hétérocycle, ou hydroxyle.

3. Composé de la revendication 1 ou la revendication 2, dans lequel X est OR_{1B}.

4. Composé de l'une quelconque des revendications 1 à 3, dans lequel R_{1B} est hydrogène ou alkyle ou aryle facultativement substitué.

5. Composé de la revendication 4, dans lequel R_{1B} est hydrogène ou alkyle.

6. Composé de l'une quelconque des revendications 1 à 3, dans lequel R₂ est hydrogène ou alkyle facultativement substitué.

7. Composé de l'une quelconque des revendications 1 à 3, dans lequel R₃ est alkyle ou halogéno.

8. Composé de la revendication 7, dans lequel R₃ est chloro.

9. Composé de l'une quelconque des revendications 1 à 3, dans lequel R₅ est alkyle ou halogéno.

10. Composé de la revendication 9, dans lequel R₅ est alkyle en C₁₋₄.

11. Composé ou sel pharmaceutiquement acceptable de celui-ci, ledit composé étant l'acide 2-((5,6-diméthylthiéno[2,3-d]pyrimidin-4-yl)thio)acétique, l'acide 2-((5-chloro-6-isopropylthiéno[2,3-d]pyrimidin-4-yl)thio)acétique, l'acide 2-((6-chloro-7-cyclopropylthiéno[3,2-d]pyrimidin-4-yl)thio)acétique, ou l'acide 2-((6-chloro-7-methylthiéno[3,2-d]pyrimidin-4-yl)thio)acétique.

12. Composition pharmaceutique comprenant un composé de l'une quelconque des revendications 1 à 11 et un excipient ou diluant pharmaceutiquement acceptable.

13. Composé de l'une quelconque des revendications 1 à 11 pour utilisation en tant que médicament pour le traitement ou la prise en charge d'une maladie ou un trouble **caractérisé par** une perte osseuse.

14. Composé de formule : 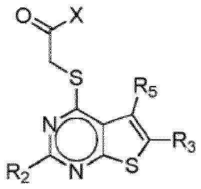 ou un sel pharmaceutiquement acceptable de celui-ci, pour utilisation tant que médicament pour le traitement ou la prise en charge d'une maladie ou un trouble **caractérisé par** une perte osseuse, dans lequel :
X est OR_{1B} ou N(R_{1B})₂,
chaque R_{1B} est indépendamment hydrogène ou alkyle, aryle, hétéroalkyle, ou hétérocycle facultativement substitué ;
R₂ est hydrogène, halogéno, -CO₂R_{2A}, -C(O)N(R_{2A})₂, -SR_{2A}, -OR_{2A}, -N(R_{2A})₂, ou alkyle, aryle, hétéroalkyle, ou hétérocycle facultativement substitué ;
chaque R_{2A} est indépendamment hydrogène ou alkyle, aryle, hétéroalkyle, ou hétérocycle facultativement substitué ;
R₃ est hydrogène, halogéno, cyano, -CO₂R_{3A}, -C(O)N(R_{3A})₂, -SR_{3A}, -OR_{3A}, -N(R_{3A})₂, ou alkyle, aryle, hétéroalkyle, ou hétérocycle facultativement substitué ;
chaque R_{3A} est indépendamment hydrogène ou alkyle, aryle, hétéroalkyle, ou hétérocycle facultativement substitué ;
R₅ est hydrogène, halogéno, -CO₂R_{5A}, -C(O)N(R_{5A})₂, -SR_{5A}, -OR_{5A}, -N(R_{5A})₂, ou alkyle, aryle, hétéroalkyle, ou hétérocycle facultativement substitué ;
chaque R_{5A} est indépendamment hydrogène ou alkyle, aryle, hétéroalkyle, ou hétérocycle facultativement substitué ; et
où « facultativement substitué » signifie facultativement substitué par l'un ou plusieurs parmi alcoxy, alkyle, amino (comprenant alkylamino, dialkylamino), aryle, acide carboxylique, cyano, halogéno, halogénoalkyle, hétérocycle, ou hydroxyle.
